(19) Europäisches Patentamt / European Patent Office / Office européen des brevets

(11) **EP 4 102 506 A1**

(12) **EUROPEAN PATENT APPLICATION**

(43) Date of publication:
**14.12.2022   Bulletin 2022/50**

(21) Application number: **22151777.4**

(22) Date of filing: **17.01.2022**

(51) International Patent Classification (IPC):
**G16B 15/30** (2019.01)    **G16B 40/20** (2019.01)
**G16C 20/30** (2019.01)    **G16C 20/50** (2019.01)
**G16C 20/70** (2019.01)

(52) Cooperative Patent Classification (CPC):
**G16B 15/30; G16B 40/20; G16C 20/30;
G16C 20/50; G16C 20/70**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority:  **26.02.2021   JP 2021031234**

(71) Applicant: **Ahead Biocomputing, Co. Ltd
Kanagawa 210-0007 (JP)**

(72) Inventors:
• **AKIYAMA, Yutaka
  TOKYO, 152-8550 (JP)**
• **OHUE, Masahito
  TOKYO, 152-8550 (JP)**

• **YANAGISAWA, Keisuke
  TOKYO, 152-8550 (JP)**
• **YOSHIKAWA, Yasushi
  TOKYO, 152-8550 (JP)**
• **SUGITA, Masatake
  TOKYO, 152-8550 (JP)**
• **FUJIE, Takuya
  TOKYO, 152-8550 (JP)**
• **SUGIYAMA, Satoshi
  TOKYO, 152-8550 (JP)**
• **MURATA, Shotaro
  TOKYO, 152-8550 (JP)**

(74) Representative: **Berggren Oy
P.O. Box 16
Eteläinen Rautatiekatu 10A
00101 Helsinki (FI)**

(54) **PREDICTION DEVICE, TRAINED MODEL GENERATION DEVICE, PREDICTION METHOD, AND TRAINED MODEL GENERATION METHOD**

(57)    A prediction device extracts each predictive feature vector expressing a feature from a peptide that is a target for membrane permeability prediction. The prediction device generates a predicted value of membrane permeability of the prediction target peptide by inputting plural predictive feature vectors into a trained model pre-trained to output a predicted value of peptide membrane permeability.

FIG.4B

EP 4 102 506 A1

**Description**

BACKGROUND

Technical Field

The present disclosure relates to a prediction device, a trained model generation device, a prediction method, a trained model generation method, a recording medium recorded with a prediction program, and a recording medium recorded with a trained model generation program.

Related Art

A molecular dynamics simulation is disclosed in Japanese Patent Application Laid-Open (JP-A) No. 2017-037378. This takes, as an initial structure in structural analysis of a biopolymer, a structure of outlier values not included in any cluster for clustering performed on plural structures in multidimensional space having all of the index dimensions included in a dimension set as coordinate axes (i.e. in claim 4).
A protein three dimensional structure prediction program disclosed in International Publication (WO) No. 2003/054743 predicts the three dimensional structure of a protein. A computer executes this protein three dimensional structure prediction program, reads in an amino acid sequence of a protein, and predicts secondary structure information. Next, the computer computes a number of amino acids to form a turn based on the secondary structure information, acquires turn structure information of a turn having a high probability of being present from the computed number of amino acids and the secondary structure information, performs prediction-reproduction of a turn, and predicts a three dimensional structure of the protein.
Moreover, Japanese National-Phase Publication No. 2020-523010 discloses a method for generating, for each patient, a set of likelihoods for a set of neoantigens for the patient by inputting a peptide sequence of each of the sets of neoantigens into a machine-learned presentation model (i.e. in claim 1).
Moreover, Japanese National-Phase Publication No. 2020-519246 discloses a method for generating a set of presentation likelihoods for a set of neoantigens by employing a processor of a computer to input numerical vectors of peptides into a deep learning presentation model (i.e. in claim 1).
Peptide drugs have recently become a focus of attention as a type of middle molecule drugs. However, there are many unclear points regarding the pharmacokinetics of peptides. In particular, peptides have a low membrane permeability, which is a measure of permeation through a cell membrane. There is accordingly a demand to predict with good accuracy whether a peptide obtained for administering as a drug has a certain degree of membrane permeability.
Technology disclosed in JP-A No. 2017-037378, WO No. 2003/054743, Japanese National-Phase Publication Nos. 2020-523010, or 2020-519246, as listed above, is technology to execute a molecular dynamics simulation of a biopolymer, technology to predict a three dimensional structure of a protein using a computer, and technology to predict a peptide that is effective as a neoantigen, and is not technology for predicting membrane permeability of a peptide. The technology in the citations above accordingly has the problem of not being able to predict peptide membrane permeability.
In consideration of the above circumstances, an object of the present disclosure is to predict membrane permeability of a peptide.

SUMMARY

A prediction device, a prediction method, and a recording medium recorded with a prediction program of a first aspect of the present disclosure are configured to extract a predictive feature vector expressing a feature from a peptide that is a target for membrane permeability prediction, to adjust such that a length of the extracted predictive feature vector is a prescribed length, and to generate a predicted value of membrane permeability for the prediction target peptide by inputting the length-adjusted predictive feature vector into a trained model pre-trained to output a predicted value of peptide membrane permeability from a feature vector expressing a feature of a peptide.
A trained model generation device, a trained model generation method, and a recording medium recorded with a trained model generation program according to a second aspect of the present disclosure are configured to extract a training feature vector expressing a feature from each of plural of training peptides, to adjust a length of each of the extracted training feature vectors for each of the plural training peptides so as to be a prescribed length, and to generate a trained model, for outputting a predicted value of peptide membrane permeability from a feature vector expressing a feature of a peptide, by executing a machine learning algorithm based on training data that is the length-adjusted training feature vectors paired with correct values of membrane permeability for the training peptides.
A prediction device, a prediction method, and a recording medium recorded with a prediction program of a third aspect of the present disclosure are configured to extract from a cyclic peptide that is a target for membrane permeability prediction each of predictive feature vectors expressing a feature for instances in which each of a plural residues contained in the cyclic peptide is at a start point of a cyclic sequence, and to generate a predicted value of membrane permeability of the prediction target cyclic peptide by inputting the plural extracted predictive feature vectors into a trained model pre-trained for outputting a predicted value of peptide membrane permeability from a feature vector expressing a feature of a cyclic peptide.

A trained model generation device, a trained model generation method, and a recording medium recorded with a trained model generation program of a fourth aspect of the present disclosure are configured to extract from each of plural training cyclic peptides a training feature vector expressing a feature for instances in which each of plural residues contained in the training cyclic peptide is at a start point of a cyclic sequence, and to generate a trained model for outputting a predicted value of cyclic peptide membrane permeability from a feature vector expressing a feature of a cyclic peptide by executing a machine learning algorithm based on training data that is the plural extracted training feature vectors for the plural training cyclic peptides paired with a correct value of membrane permeability for the respective training cyclic peptide.

A prediction device, a prediction method, and a recording medium recorded with a prediction program of a fifth aspect of the present disclosure are a prediction device, a prediction method, and a recording medium recorded with a prediction program for computing a predicted value of membrane permeability of a peptide permeating through a membrane region representing a cell membrane, a first solvent region representing a solvent adjacent to one side of the membrane region, and a second solvent region representing a solvent adjacent to another side of the membrane region. Based on a result of simulation of a peptide permeating through the first solvent region, the membrane region, and the second solvent region, a free energy $G(z)$ of the peptide is computed for each reaction coordinate $z$ expressing a position of the peptide in a region including the first solvent region, the membrane region, and the second solvent region and expressing a position of the peptide in a direction of an axis perpendicular to a membrane surface of the membrane region. At each of the reaction coordinates $z$, a difference $\Delta G(z)$ is computed between a minimum value $G_{min}$ out of the free energies $G(z)$ of the peptide computed for the reaction coordinates $z$ and the free energy $G(z)$ of the peptide at the reaction coordinate $z$. A local diffusion coefficient $D(z)$ is computed at each of the reaction coordinates $z$, and a value $R(z)$ expressing a local resistance of the peptide at the reaction coordinate $z$ is computed based on the difference $\Delta G(z)$ computed for each of the reaction coordinates $z$ and based on the local diffusion coefficient $D(z)$ computed for each of the reaction coordinates $z$. A predicted value of membrane permeability is computed for the peptide based on the value $R(z)$ expressing the local resistances computed for each of the reaction coordinates $z$.

A prediction device, a prediction method, and a recording medium recorded with a prediction program according to a sixth aspect of the present disclosure are a prediction device, a prediction method, and a recording medium recorded with a prediction program for simulating dynamics of a peptide permeating through a membrane region representing a cell membrane, a first solvent region representing a solvent adjacent to one side of the membrane region, and a second solvent region representing a solvent adjacent to another side of the membrane region. An initial conformation of the peptide is set according to a relative substance permittivity in the first solvent region for simulation of the peptide permeating a segment spanning from the first solvent region to a vicinity of a lipid molecule join positioned further toward a membrane center side than a boundary between the first solvent region and the membrane region. An initial conformation of the peptide is set according to a relative substance permittivity in the membrane region for simulation of the peptide permeating a segment spanning from the vicinity of the join to past a region of a membrane central zone representing a central area of the membrane region. Dynamics of the peptide are simulated according to the set initial conformation of the peptide and a series of initial conformations are set at respective regions using an umbrella sampling method based on a result of simulation obtained. The dynamics of the peptide are simulated according to an umbrella sampling method based on the series of initial conformations set for each of the regions, and membrane permeability of the peptide is predicted based on a result of simulation based on an umbrella sampling.

A prediction device, a prediction method, and a recording medium recorded with a prediction program according to a seventh aspect of the present disclosure are a prediction device, a prediction method, and a recording medium recorded with a prediction program for simulating dynamics of a peptide permeating through a membrane region representing a cell membrane, a first solvent region representing a solvent adjacent to one side of the membrane region, and a second solvent region representing a solvent adjacent to another side of the membrane region. When simulating permeation of the peptide using an umbrella sampling method, a spacing between restraint positions of the peptide is set so as to be finer the closer a region is to a membrane central zone representing a central area of the membrane region. Dynamics of the peptide are simulated using an umbrella sampling method according to the spacing set between the restraint positions, and membrane permeability of the peptide is predicted based on a result of the simulation.

A prediction device, a prediction method, and a recording medium recorded with a prediction program according to an eighth aspect of the present disclosure are configured to generate a first membrane permeability value expressing peptide membrane permeability by simulating dynamics of the peptide permeating through a membrane region representing a cell membrane, a first solvent region representing a solvent adjacent to one side of the membrane region, and a second solvent region representing a solvent adjacent to another side of the membrane region, to generate a second membrane permeability value expressing membrane permeability of the peptide by extracting a predictive feature vector expressing a feature from the peptide and inputting the predictive feature vector into a trained model previously subjected to machine learning, and to compute a predicted value of membrane permeability of the peptide by consolidating the generated first membrane permeability value with the generated second membrane permeability value.

A trained model generation device, a trained model generation method, and a recording medium recorded with a trained model generation program according to a ninth aspect of the present disclosure are configured to generate a predicted value of membrane permeability expressing membrane permeability of a peptide by simulating dynamics of the peptide permeating through a membrane region representing a cell membrane, a first solvent region representing a solvent adjacent to one side of the membrane region, and a second solvent region representing a solvent adjacent to another side of the membrane region, to generate simulation-derived training data expressed by the obtained predicted value of peptide membrane permeability paired with a feature vector generated from a 3D descriptor obtained from a tertiary structure of the peptide at each location in the membrane region, the first solvent region, or the second solvent region, and to generate a trained model, for outputting a predicted value of the membrane permeability from the feature vector, by executing a machine learning algorithm based on training data including the generated simulation-derived training data.

A trained model generation device, a trained model generation method, and a recording medium recorded with a trained model generation program according to a tenth aspect of the present disclosure are configured to extract a first training feature vector expressing a feature from each of plural training cyclic peptides, to generate plural second training feature vectors for each of the extracted first training feature vectors by cyclically shifting elements of the first training feature vectors, and to generate training data expressed by the first training feature vector and the plural second training feature vectors paired with a correct value of membrane permeability of the respective training cyclic peptide, and to generate a trained model, for outputting a predicted value of membrane permeability of a cyclic peptide from a feature vector expressing a feature of a cyclic peptide, by executing a machine learning algorithm based on the plural generated training data.

A trained model generation device, a trained model generation method, and recording medium recorded with a trained model generation program according to an eleventh aspect of the present disclosure are configured to, based on training data expressed by a training feature vector expressing a feature extracted for each of plural training cyclic peptides paired with correct values of membrane permeability for the plural training cyclic peptides, to generate a trained convolutional neural network model for outputting a predicted value of membrane permeability of a cyclic peptide from a feature vector expressing a feature of a cyclic peptide by executing a machine learning algorithm using a convolutional neural network model including a both-end-adjacency layer in which elements at both ends of the training feature vectors are placed adjacent to one another.

A prediction device, a prediction method, and a recording medium recorded with a prediction program according to a twelfth aspect of the present disclosure are configured to extract a predictive feature vector expressing a feature from a cyclic peptide that is a target for membrane permeability prediction, and to generate a predicted value of membrane permeability of the prediction target peptide by inputting the extracted predictive feature vector into a trained convolutional neural network model for outputting a predicted value of membrane permeability of a peptide from the feature vector that is a trained convolutional neural network model including a both-end-adjacency layer in which elements at both ends of a feature vector expressing a feature of a cyclic peptide are placed adjacent to one another.

The present disclosure obtains the advantageous effect of being able to predict membrane permeability of a peptide.

The object and advantages of the invention will be realized and attained by means of the elements and combinations particularly pointed out in the claims.

It is to be understood that both the foregoing general description and the following detailed description are exemplary and explanatory and are not restrictive of the invention.

BRIEF DESCRIPTION OF THE DRAWINGS

Fig. 1 is a block diagram illustrating a prediction device according to a first exemplary embodiment.
Fig. 2 is a diagram illustrating an example of data stored in a data storage section 12.
Fig. 3A is a diagram to explain a cyclic peptide.
Fig. 3B is a diagram to explain a structure of a cyclic peptide.
Fig. 4A is a diagram illustrating an example of training data stored in a training data storage section 16.
Fig. 4B is a diagram to explain a trained model.
Fig. 5 is a diagram illustrating a computer to implement a prediction device according to the first exemplary embodiment.

Fig. 6 is a diagram illustrating an example of a trained model generation processing routine executed in a prediction device according to the first exemplary embodiment.

Fig. 7 is a diagram illustrating an example of a prediction processing routine executed in a prediction device according to the first exemplary embodiment.

Fig. 8 is a block diagram illustrating a prediction device according to a second exemplary embodiment.

Fig. 9 is a diagram illustrating an example of a trained model generation processing routine executed in a prediction device according to the second exemplary embodiment.

Fig. 10 is a diagram illustrating an example of a prediction processing routine executed in a prediction device according to the second exemplary embodiment.

Fig. 11 is a block diagram illustrating a prediction device according to a third exemplary embodiment.

Fig. 12 is a diagram to explain generation of second training feature vectors.

Fig. 13 is a configuration diagram of a conventional convolutional neural network model.

Fig. 14 is a configuration diagram of a convolutional neural network model of a fourth exemplary embodiment.

Fig. 15 is a block diagram illustrating a prediction device according to a fifth exemplary embodiment.

Fig. 16 is a diagram to explain a manner by which a peptide permeates through a cell membrane.

Fig. 17 is a diagram to explain each location in a molecular dynamics simulation of the fifth exemplary embodiment.

Fig. 18 is a diagram to explain restraint positions of a peptide in a replica exchange umbrella sampling method.

Fig. 19 is a diagram to explain a minimum value $G_{min}$ out of free energy $G(z)$ of a peptide.

Fig. 20 is a diagram illustrating an example of a simulation processing routine executed in a prediction device according to the fifth exemplary embodiment.

Fig. 21 is a block diagram illustrating a prediction device according to a sixth exemplary embodiment.

Fig. 22 is a block diagram illustrating a prediction device according to a seventh exemplary embodiment.

DETAILED DESCRIPTION

Detailed explanation follows regarding exemplary embodiments of the present invention, with reference to the drawings.

First Exemplary Embodiment

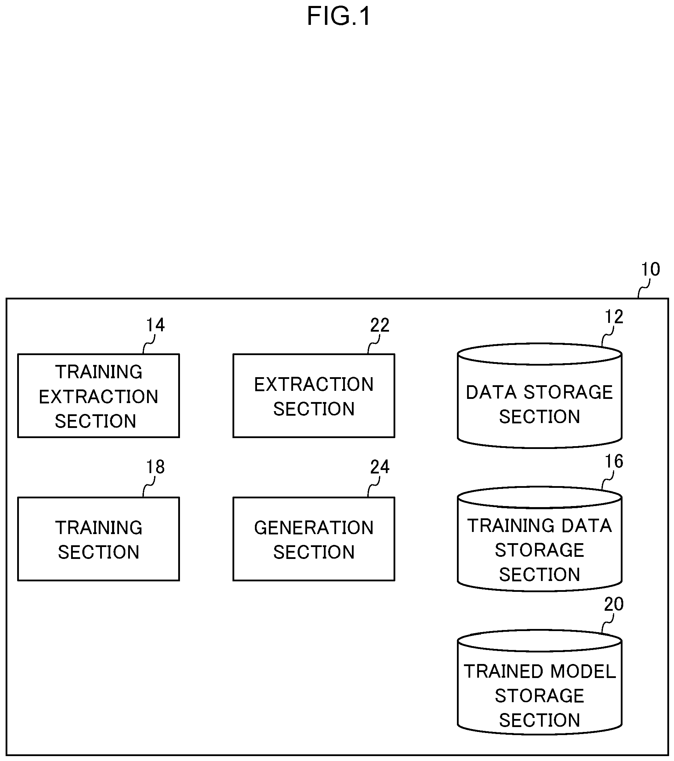

Fig. 1 is a block diagram illustrating an example of a configuration of a prediction device 10 according to a first exemplary embodiment. As illustrated in Fig. 1, in terms of functionality, the prediction device 10 includes a data storage section 12, a training extraction section 14, a training data storage section 16, a training section 18, a trained model storage section 20, an extraction section 22, and a generation section 24.

The prediction device 10 of the present exemplary embodiment predicts membrane permeability of cyclic peptides.

Training peptide information expressing cyclic peptides used for training and correct values for membrane permeability of these training cyclic peptides are stored associated with each other in the data storage section 12. Note that the peptide information is information including at least one type of information from out of a chemical formula of the peptide, SMILES notation of the peptide, a primary structure of the peptide, a secondary structure of the peptide, a tertiary structure of the peptide, or a quaternary structure the peptide.

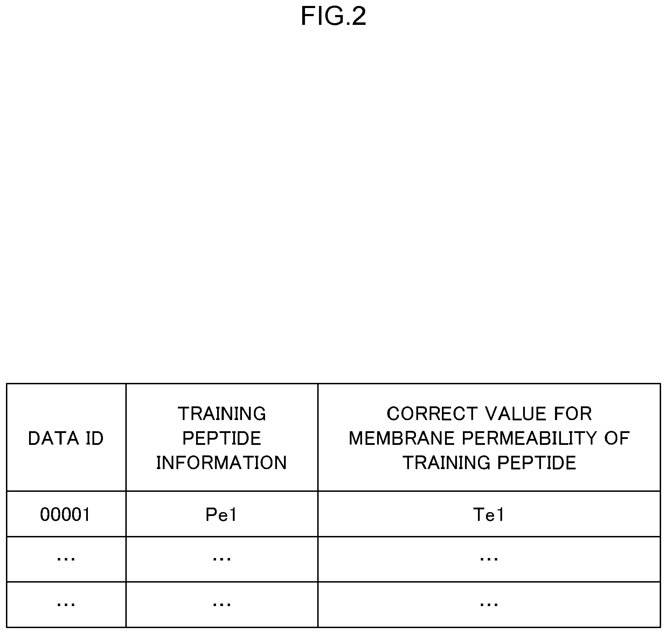

The correct values for membrane permeability of the training cyclic peptides are, for example, data obtained by performing known experiments on the training cyclic peptides. Fig. 2 illustrates an example of data stored in the data storage section 12. As illustrated in Fig. 2, the training peptide information and the correct values for membrane permeability of the training cyclic peptides are stored associated with each other in the data storage section 12.

The training extraction section 14 extracts training feature vectors expressing features of cyclic peptides from the plural training peptide information stored in the data storage section 12. Note that the feature vectors are extracted from the peptide information using a known method.

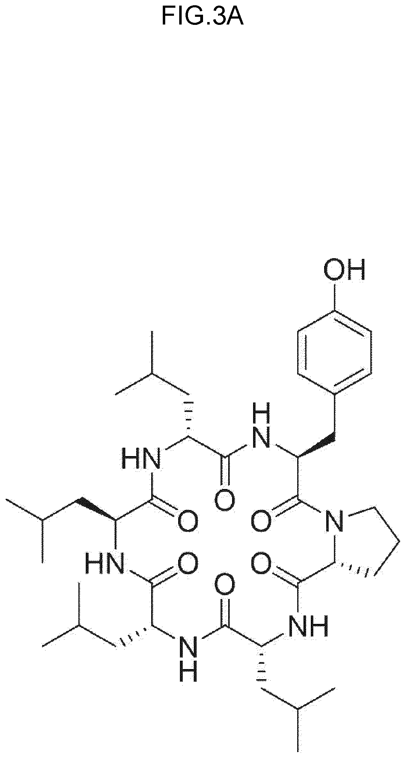

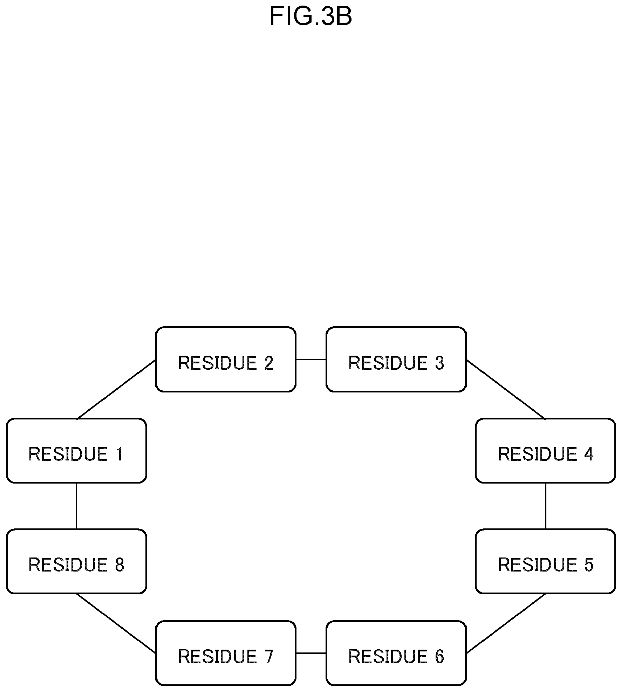

Fig. 3A and Fig. 3B are diagrams for explaining the structure of a cyclic peptide. Fig. 3A is a diagram illustrating an example of a cyclic peptide. The cyclic peptide illustrated in Fig. 3A includes plural residues, and a ring is formed by these residues. Fig. 3B schematically illustrates a configuration of a cyclic peptide. When configuring the feature vectors of the cyclic peptide by extracting an overall feature vector of the cyclic peptide and a feature vector of each residue from a cyclic peptide such as that illustrated in Fig. 3B, the feature vector configuration differs depending on which residue is at a start point of the cyclic sequence.

For example, for a feature vector configuration in which the residue 1 illustrated in Fig. 3B is at the start point of the cyclic sequence, the feature vector [F1, F2,... F8] is configured with the feature value F1 extracted from the residue 1 at the start point. On the other hand, for a feature vector configuration in which the residue 8 is at the start point of the cyclic sequence, the feature vector [F8, F1, F2... F7] is extracted with the feature value F8 extracted from the residue 8 at the start point.

Thus, even if the cyclic peptides are the same, the feature vectors will be different in cases in which the residue at the start point of the cyclic sequence is different. The membrane permeability of cyclic peptides is not able to be appropriately predicted for such cases.

To address this, in the present exemplary embodiment, the respective feature vectors are extracted for instances in which each of the plural residues contained in the cyclic peptide is at the start point of the cyclic sequence, and the membrane permeability is predicted based on these plural feature vectors.

Specifically, from peptide information for each of plural items of training cyclic peptides, the training extraction section 14 extracts feature vectors expressing features for instances in which each of the plural residues contained in the training cyclic peptide is at the start point of the cyclic sequence.

For example, the training extraction section 14 extracts a feature vector 1 for an instance in which the residue 1 illustrated in Fig. 3B is at the start point of the cyclic sequence, extracts a feature vector 2 for an instance in which the residue 2 is at the start point of the cyclic sequence, and so on until it extracts a feature vector 8 for an instance in which the residue 8 is at the start point of the cyclic sequence.

The training extraction section 14 sets each single extracted feature vector as a single training feature vector. Thus, a set of feature vectors extracted from a single training cyclic peptide corresponds to a training feature vector set.

For each of the plural training cyclic peptides, the training extraction section 14 associates the set of training feature vectors with a correct value for membrane permeability of the training peptide, and stores these in the training data storage section 16.

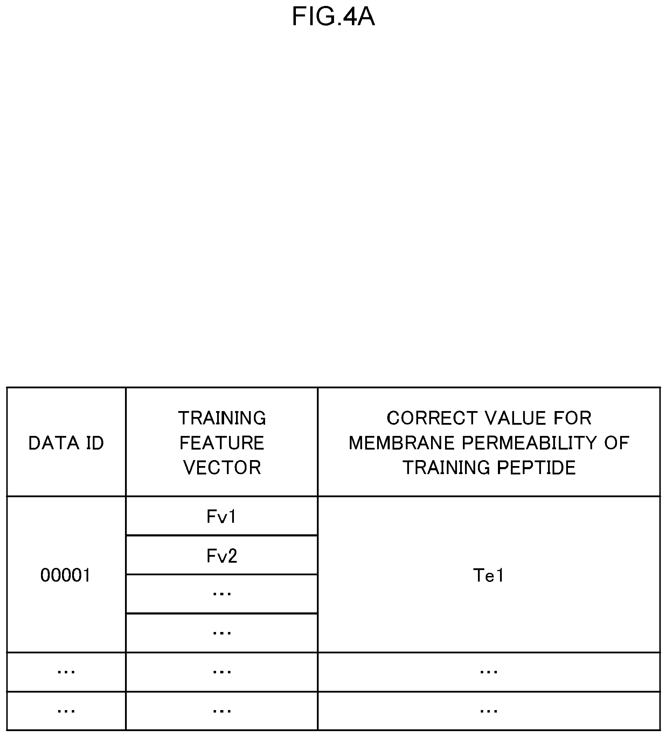

Plural training data are stored in the training data storage section 16. A single item of training data is training feature vectors paired with a correct value for membrane permeability of the training peptide. Fig. 4A illustrates an example of the training data stored in the training data storage section 16. As illustrated in Fig. 4A, the training feature vectors and the correct value for membrane permeability of the training peptides are stored associated with each other in the training data storage section 16. This training data is employed to generate a trained model, described later. Note that the plural training feature vectors Fv1, Fv2, etc. in the example in Fig. 4A are training feature vectors obtained by employing different start points for the cyclic sequence.

The training section 18 generates a trained model, for outputting a predicted value for peptide membrane permeability from feature vectors, by executing a known supervised machine learning algorithm based on the plural training data stored in the training data storage section 16. The training section 18 then stores the trained model in the trained model storage section 20. Note that the trained model itself is a known model, and may for example be a neural network model, a support vector machine, a logistic regression model, or the like. Note that neural network models include deep neural network models obtained by deep learning.

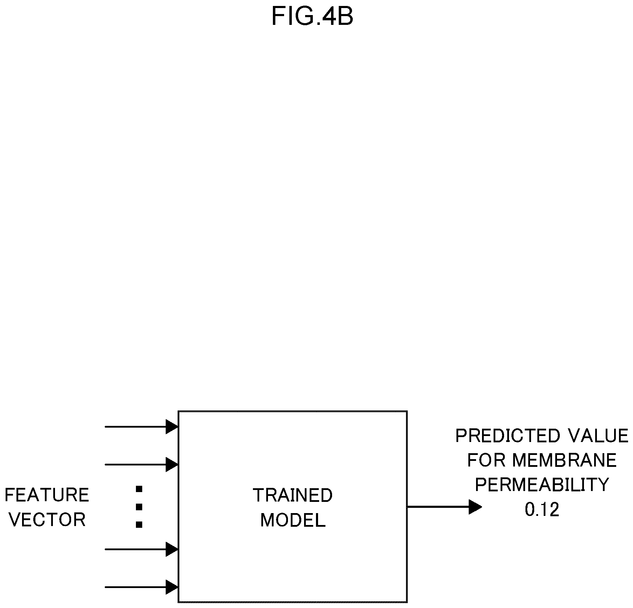

Fig. 4B is a diagram for explaining a trained model. As illustrated in Fig. 4B, when feature vectors extracted from a cyclic peptide that is a target for membrane permeability prediction are input into the trained model, a predicted value is output for the membrane permeability of the prediction target cyclic peptide.

Note that as described below, plural feature vectors are also extracted from the cyclic peptide that is the target for membrane permeability prediction by employing different start points for the cyclic sequence. By inputting each of these plural feature vectors into the trained model a predicted value of membrane permeability is obtained corresponding to each of the plural feature vectors.

The trained model generated by the training section 18 is stored in the trained model storage section 20. Note that the trained model is data in which a structure and trained parameters of a model are associated with each other.

The extraction section 22 extracts feature vectors expressing features from the cyclic peptide that is the target for membrane permeability prediction. Specifically, from the peptide information regarding the cyclic peptide that is the target for membrane permeability prediction, the extraction section 22 extracts respective feature vectors (hereafter referred to as predictive feature vectors) expressing features for instances in which each of the plural residues contained in the cyclic peptide is at the start point of the cyclic sequence.

The generation section 24 generates a predicted value of membrane permeability for the prediction target cyclic peptide by inputting the plural predictive feature vectors obtained by the extraction section 22 into the trained model stored in the trained model storage section 20.

Specifically, the generation section 24 generates respective predicted values for membrane permeability of the prediction target peptide by inputting each of plural predictive feature vectors obtained by the extraction section 22 into the trained model. Note that a single predicted value corresponds to a single predictive feature vector. The generation section 24 then generates a representative value for the plural predicted values and sets the representative value as the membrane permeability of the peptide that is the prediction target. For example, the generation section 24 may generate an average value of the plural predicted values as the representative value. Alternatively, the generation section 24 may generate a maximum value or a minimum value of the plural predicted values as the representative value.

Note that either the plural predicted values or the representative value for membrane permeability generated by the generation section 24 may be displayed on a display section (not illustrated in the drawings).

In this manner, the prediction device 10 of the first exemplary embodiment extracts respective feature vectors for instances in which each of the plural residues contained in a cyclic peptide is at the start point of the cyclic sequence, and predicts membrane permeability based on these plural feature vectors. This obtains plural feature vectors in consideration of rotational symmetry of the cyclic peptide, and enables membrane permeability of the cyclic peptide to be predicted in an appropriate manner based on these feature vectors.

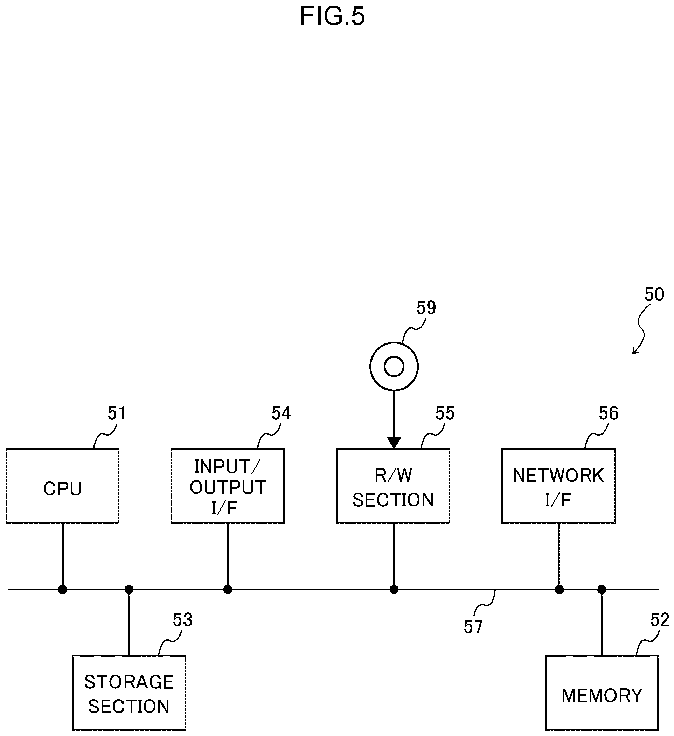

The prediction device 10 may for example be implemented by a computer 50 such as that illustrated in Fig. 5. The computer 50 implementing the prediction device 10 includes a CPU 51, memory 52 serving as a temporary storage area, and a non-volatile storage section 53. The computer 50 also includes an input/output interface (I/F) 54 to which an input/output device or the like (not illustrated in the drawings) is connected, and a read/write (R/W) section 55 that controls reading of data from, and writing of data to, a recording medium 59. The computer 50 also includes a network I/F 56 connected to a network such as the internet. The CPU 51, the memory 52, the storage section 53, the input/output I/F 54, the R/W section 55, and the network I/F 56 are connected to each other through a bus 57.

The storage section 53 may be implemented by a hard disk drive (HDD), a solid state drive (SSD), flash memory, or the like. The storage section 53 serves as a storage medium and is stored with a program causing the computer 50 to function. The CPU 51 reads the program from the storage section 53, expands the program in the memory 52, and sequentially execute processes in the program.

Next, explanation follows regarding operation of the prediction device 10 of the first exemplary embodiment.

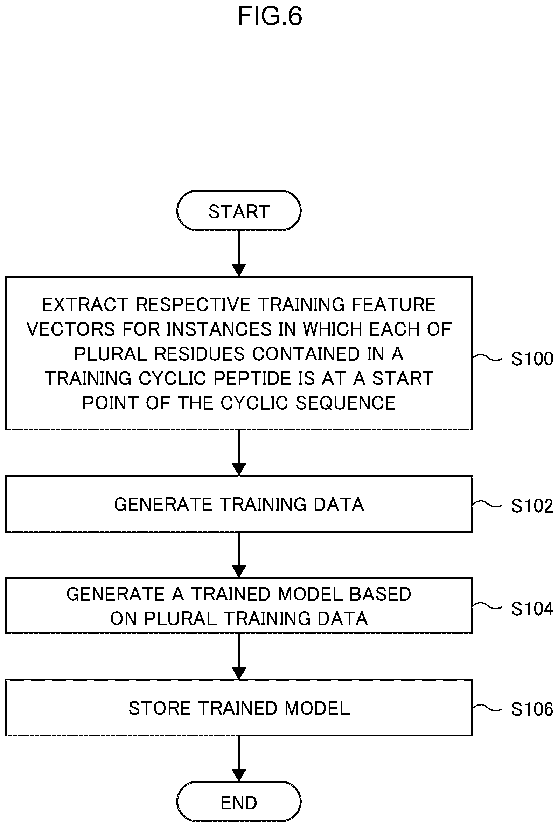

On receiving an instruction signal indicating an instruction to perform trained model generation processing, the prediction device 10 executes a trained model generation processing routine as illustrated in Fig. 6.

At step S100, the training extraction section 14 extracts, from each of the peptide information for plural training cyclic peptides, the training feature vectors expressing features for the instances in which each of the plural residues contained in a training cyclic peptide is at the start point of the cyclic sequence.

At step S102, the training extraction section 14 associates the set of training feature vectors extracted at step S100 with a correct value for membrane permeability of the training cyclic peptide to generate training data, and temporarily stores this training data in the training data storage section 16.

At step S104, the training section 18 generates a trained model, for outputting a predicted value for peptide membrane permeability from feature vectors, by executing a known supervised machine learning algorithm based on the plural training data stored in the training data storage section 16.

At step S106, the training section 18 stores the trained model generated at step S104 in the trained model storage section 20.

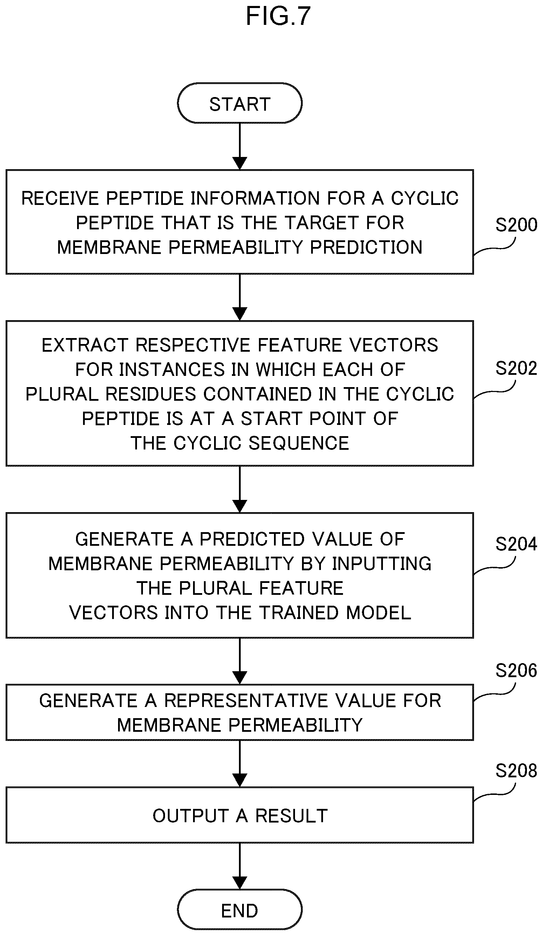

When the trained model has been stored in the trained model storage section 20 and the peptide information for the target for membrane permeability prediction has been input to the prediction device 10, the prediction device 10 executes the prediction processing routine illustrated in Fig. 7.

At step S200, the extraction section 22 receives the peptide information for the target for membrane permeability prediction.

At step S202, from the peptide information received at step S200, the extraction section 22 extracts respective predictive feature vectors expressing features for instances in which each of the plural residues contained in the cyclic peptide is at the start point of the cyclic sequence.

At step S204, the generation section 24 generates plural predicted values of membrane permeability for the prediction target peptide by inputting each of the plural predictive feature vectors extracted at step S202 into the trained model stored in the trained model storage section 20.

At step S206, the generation section 24 generates a representative value from the plural predicted values generated at step S204.

At step S208, the generation section 24 outputs the representative value of the predicted values of membrane permeability generated at step S206 as a result.

As described in detail above, the prediction device of the first exemplary embodiment extracts from each of plural training cyclic peptides a set of training feature vectors expressing features for instances in which each of plural residues contained in the training cyclic peptide is at the start point of the cyclic sequence. Then, for each of the plural training cyclic peptides, the prediction device executes a machine learning algorithm based on the training data of the extracted plural training feature vectors paired with correct values for the membrane permeability of the training cyclic peptides, so as to generate a trained model for outputting a predicted value of membrane permeability for a cyclic peptide from feature vectors expressing cyclic peptide features. This enables a trained model to be obtained for predicting the membrane permeability of cyclic peptides. Note that the trained model is trained based on training feature vectors for instances in which each of the plural residues is at the start point of the cyclic sequence, and so the model is suited to predicting the membrane permeability of cyclic peptides.

Moreover, the prediction device of the first exemplary embodiment extracts from a cyclic peptide that is the target for membrane permeability prediction respective feature vectors expressing features for instances in which each of plural residues contained in the cyclic peptide is at the start point of the cyclic sequence. The prediction device then generates a predicted value of membrane permeability for the prediction target cyclic peptide by inputting the plural feature vectors into the trained model. This enables the membrane permeability of the cyclic peptide to be predicted.

Specifically, as described previously, the trained model is trained based on the training feature vectors for instances in which each of the plural residues is at the start point of the cyclic sequence, and so the model is suited to predicting the membrane permeability of cyclic peptides. This enables predicted values for membrane permeability to be generated in consideration of the cyclic peptide structure.

Second Exemplary Embodiment

Next, explanation follows regarding a second exemplary embodiment. A prediction device of the second exemplary embodiment differs from the first exemplary embodiment in that lengths of the plural feature vectors are aligned. Note that although an example of a case applied to cyclic peptides as the target has been described in the first exemplary embodiment, there is no limitation to cyclic peptides in the second exemplary embodiment, and linear peptides may be the target. Moreover, similar portions in the configuration of the prediction device according to the second exemplary embodiment to those of the prediction device of the first exemplary embodiment are allocated the same reference numerals, and explanation thereof is omitted.

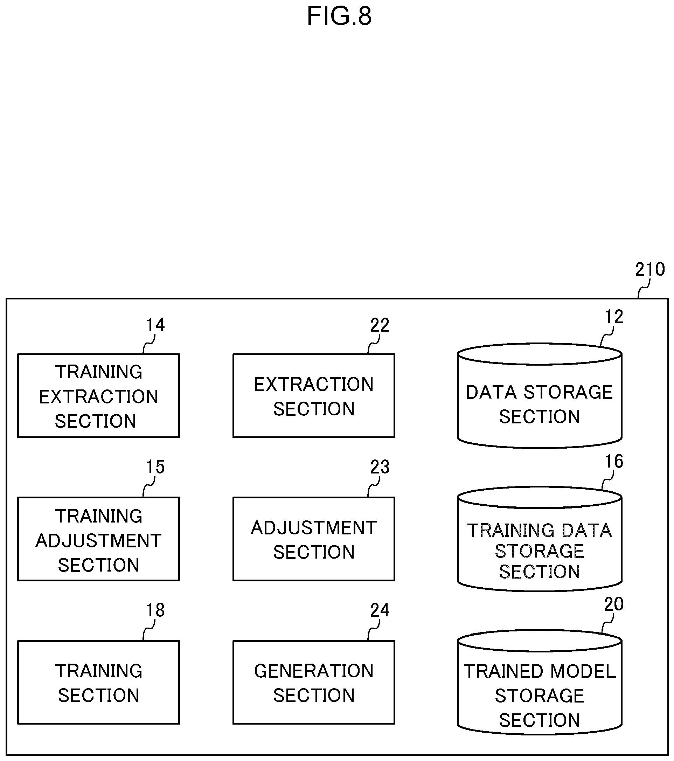

Fig. 8 is a block diagram illustrating an example of a configuration of a prediction device 210 according to the second exemplary embodiment. As illustrated in Fig. 8, in terms of functionality, the prediction device 210 includes the data storage section 12, the training extraction section 14, the training data storage section 16, a training adjustment section 15, the training section 18, the trained model storage section 20, the extraction section 22, an adjustment section 23, and the generation section 24.

The training adjustment section 15 performs adjustment such that the respective lengths of the training feature vectors of the plural training peptides extracted by the training extraction section 14 become a prescribed length.

The peptides include plural residues. Thus the length of the feature vectors differs between peptides that have a different number of residues. Specifically, the number of feature vector elements correspond to the number of residues, and so the length of the feature vectors differs between peptides that have a different number of residues. Note that feature vectors input into a trained model such as a neural network model are preferably uniform. For example, in cases in which the number of feature vector elements is ten, an action is required to make it such that there is also a corresponding ten nodes in the input layer of the neural network model, an example of a trained model.

Thus, in cases in which the lengths of the feature vectors extracted from each of the plural peptides differ, unless some appropriate measure is taken, a trained model employing a machine learning algorithm such a neural network model cannot be built, or the peptide membrane permeability cannot be predicted using such a trained model.

To address this, in the prediction device of the second exemplary embodiment, the lengths of the feature vectors extracted from the peptides are aligned, thereby enabling training to be performed using a machine learning algorithm that employs these feature vectors. Furthermore, the peptide membrane permeability can be predicted using a trained model obtained by training.

Specifically, for example, the training adjustment section 15 identifies the training feature vector with the maximum length from out of the plural training feature vectors, and performs adjustment such that the lengths of the plural other training feature vectors become this maximum length. Alternatively, for example, the training adjustment section 15 may perform adjustment such that the respective lengths of the plural training feature vectors become a prescribed length. Note that the prescribed length in such cases may be preset by a user.

For example, the training adjustment section 15 may align the length of the training feature vectors by converting using a known padding method. A padding method is a method in which a vacant location of a target is filled with a substitute value or the like. Thus, for example, in the case of a training feature vector [0.13, 0.45, 0.82] with a length of three, if the prescribed length is five then the training adjustment section 15 may use a padding method so as to generate a training feature vector with a length of five such as [0.00, 0.13, 0.45, 0.82, 0.00]. Note that when adjusting the lengths of the training feature vectors, the training adjustment section 15 may add an element containing information about the length pre-adjustment, such as the number of residues prior to length adjustment.

Alternatively, for example, the training adjustment section 15 may align the lengths of the training feature vectors by conversion using a linear interpolation method. Specifically, the training adjustment section 15 may compute a feature value x', this being an element of a training feature vector, using the following Equation (1).

$$j' = \left\lceil \frac{(k-1)(j-1)}{(m-1)} \right\rceil + 1$$

$$x'_j = \frac{(n-1)(j-1)-(m-1)(j'-1)}{(m-1)(k-1)}\left(x_{j'+1} - x_{j'}\right) + x_{j'}$$

$$(1)$$

Wherein $x_i$ is a feature value of a residue position i of a peptide with residue length k ($1 \le i \le k$); and
$x'_j$ is a $j^{th}$ feature value of sequence length m after interpolation ($1 \le j \le m$)

The training adjustment section 15 converts a training feature vector with a length k obtained from a peptide with a residue length k into a training feature vector with a length m according to the Equation (1). Note that $x_i$ is a feature value at the position of an $i^{th}$ element of a training feature vector x prior to conversion, and $x'_j$ is a feature value at the position of an $j^{th}$ element of a training feature vector x' after conversion. The lengths of plural training feature vectors are aligned in this manner.

The training adjustment section 15 then associates the training feature vectors having aligned lengths with the correct values for membrane permeability of the corresponding training peptides, and stores these in the training data storage section 16.

There are plural training data stored in the training data storage section 16.

The training section 18 generates a trained model, for outputting a predicted value for peptide membrane permeability from feature vectors, by executing a known supervised machine learning algorithm based on the plural training data stored in the training data storage section 16. The training section 18 then stores the trained model in the trained model storage section 20.

The trained model generated by the training section 18 is stored in the trained model storage section 20.

The extraction section 22 extracts predictive feature vectors expressing features from the membrane permeability prediction target cyclic peptide.

The adjustment section 23 performs adjustment such that the lengths of the predictive feature vectors extracted by the extraction section 22 are the same prescribed length as those in the training data. Specifically, the adjustment section 23 adjusts the lengths of the predictive feature vectors using a similar method to the training adjustment section 15 as described above.

The generation section 24 generates a predicted value for membrane permeability of the prediction target peptide by inputting the predictive feature vectors with their lengths adjusted by the adjustment section 23 into the trained model stored in the trained model storage section 20.

Note that the predicted values for membrane permeability generated by the generation section 24 are displayed on a display section (not illustrated in the drawings).

Next, explanation follows regarding operation of the prediction device 210 of the second exemplary embodiment.

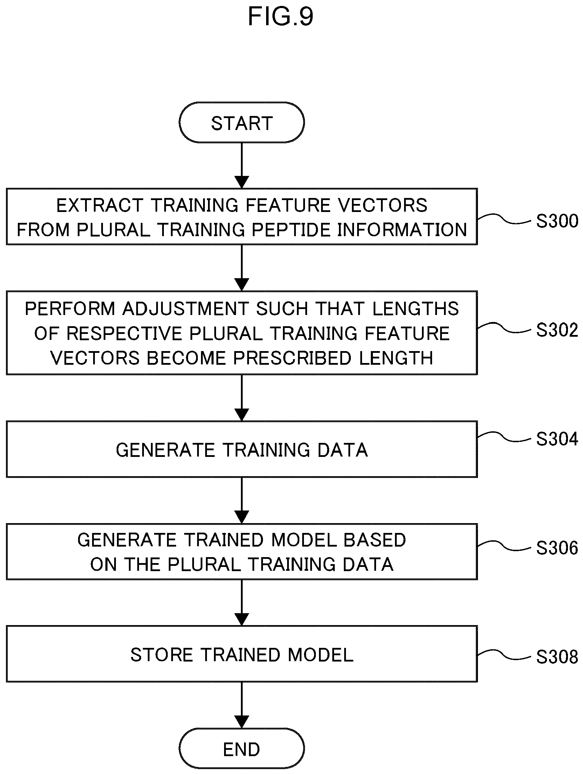

On receiving an instruction signal indicating an instruction to perform trained model generation processing, the prediction device 210 executes the trained model generation processing routine illustrated in Fig. 9.

At step S300, the training extraction section 14 extracts training feature vectors expressing features of the training peptide from the plural training peptide information stored in the data storage section 12.

At step S302, the training adjustment section 15 performs adjustment such that the respective lengths of the training feature vectors for the plural training peptides extracted at step S300 become a prescribed length.

At step S304, the training adjustment section 15 associates the training feature vectors having lengths aligned at step S302 with respective correct values for membrane permeability of the training peptides and generates training data to be temporarily stored in the training data storage section 16.

At step S306, the training section 18 generates a trained model, for outputting a predicted value for peptide membrane permeability from feature vectors expressing peptide features, by executing a known supervised machine learning algorithm based on the plural training data stored in the training data storage section 16.

At step S308, the training section 18 stores the trained model generated at step S306 in the trained model storage section 20.

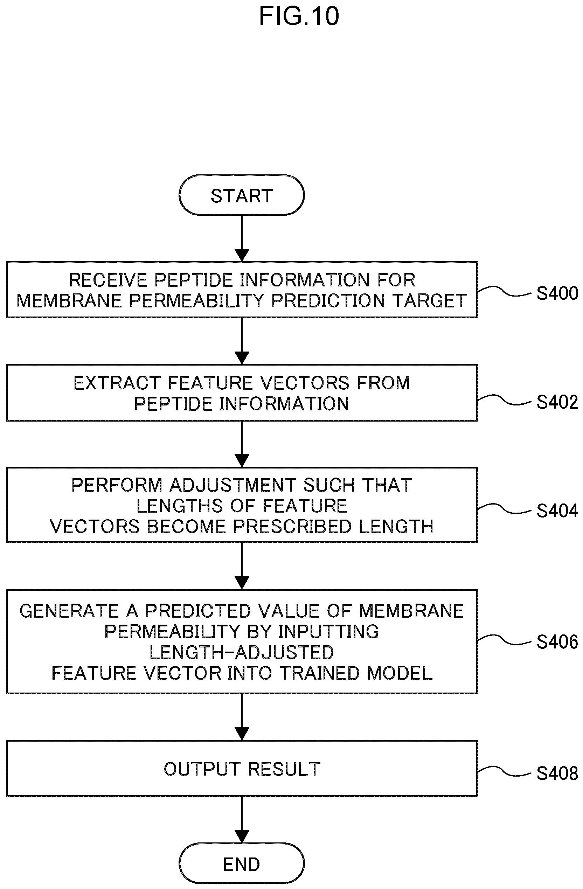

When the trained model has been stored in the trained model storage section 20, and the peptide information for the target for membrane permeability prediction has been input to the prediction device 210, the prediction device 210 executes the prediction processing routine illustrated in Fig. 10.

At step S400, the extraction section 22 receives the peptide information for the target for membrane permeability prediction.

At step S402, the extraction section 22 extracts predictive feature vectors from the peptide information received at step S400.

At step S404, the adjustment section 23 performs adjustment such that the lengths of the predictive feature vectors extracted at step S402 become the prescribed length.

At step S406, the generation section 24 generates a predicted value of membrane permeability for the prediction target peptide by inputting the predictive feature vectors having lengths adjusted at step S404 into the trained model stored in the trained model storage section 20.

At step S408, the generation section 24 outputs the predicted value for membrane permeability generated at step S406 as a result.

As described in detail above, the prediction device of the second exemplary embodiment performs adjustment such that the respective lengths of the training feature vectors for the plural training peptides become the prescribed length. The prediction device then generates a trained model, for outputting a predicted value for peptide membrane permeability from feature vectors extracted from peptides, by executing a machine learning algorithm based on the training data in which the length-adjusted training feature vectors are paired with the respective correct values for membrane permeability of the training peptides. Thus a trained model can be obtained for predicting peptide membrane permeability, even in cases in which peptides are configured from plural residues having a different number of residues.

Moreover, the prediction device of the second exemplary embodiment generates a predicted value for membrane permeability of a prediction target peptide by adjusting the length of feature vectors extracted from the peptide that is the target for membrane permeability prediction so as to become the prescribed length, and inputting the length-adjusted feature vectors into the trained model. This enables the peptide membrane permeability to be predicted even in cases in which peptides are configured from plural residues having a different number of residues.

Third Exemplary Embodiment

Next, explanation follows regarding a third exemplary embodiment. A prediction device of the third exemplary embodiment differs from the first and second exemplary embodiments in respect that the training data is augmented by data augmentation that focuses on the structural properties of a cyclic peptide, and a trained model is generated based on this augmented training data. Note that similar portions in the configuration of the prediction device according to the third exemplary embodiment to those of the prediction devices of the first and second exemplary embodiments are allocated the same reference numerals, and explanation thereof is omitted.

When augmenting the training feature vectors, the prediction device of the third exemplary embodiment performs a similar length adjustment to that in the second exemplary embodiment, and then cyclically shifts elements of the training feature vectors so as to generate plural training feature vectors. This enables the training data to be augmented while considering structural characteristic of the cyclic peptides.

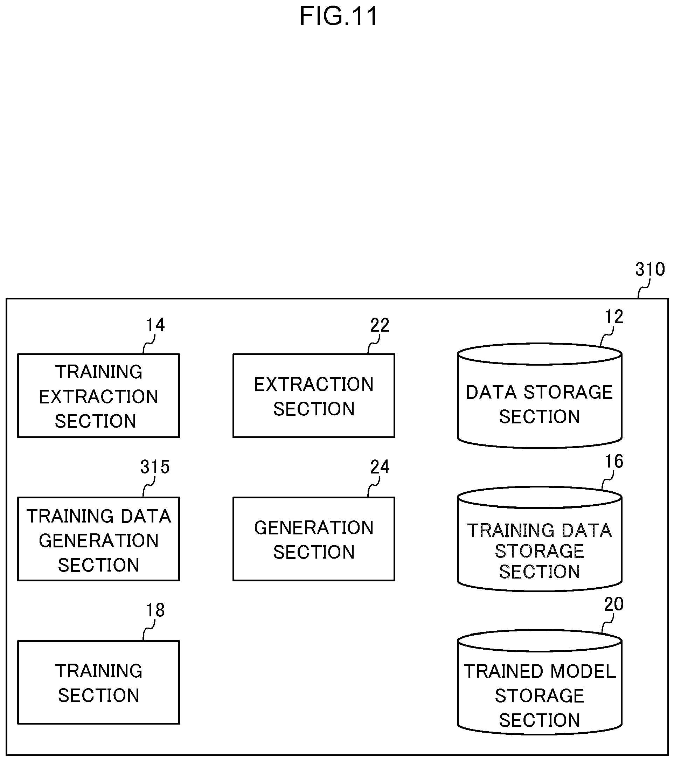

Fig. 11 is a block diagram illustrating an example of a configuration of a prediction device 310 according to the third exemplary embodiment. As illustrated in Fig. 11, in terms of functionality the prediction device 310 includes the data storage section 12, the training extraction section 14, the training data storage section 16, a training data generation section 315, the training section 18, the trained model storage section 20, the extraction section 22, and the generation section 24.

The training extraction section 14 of the third exemplary embodiment extracts a set of first training feature vectors expressing features of a training cyclic peptide from out of each of the plural peptide information regarding training cyclic peptides.

Specifically, first, the training data generation section 315 aligns the lengths of the plural first training feature vectors to a prescribed length, similarly to in the second exemplary embodiment. Next, for each of the first training feature vectors included in the first training feature vector set extracted by the training extraction section 14, the training data generation section 315 cyclically shifts elements of the first training feature vectors to generate a set of second training feature vectors.

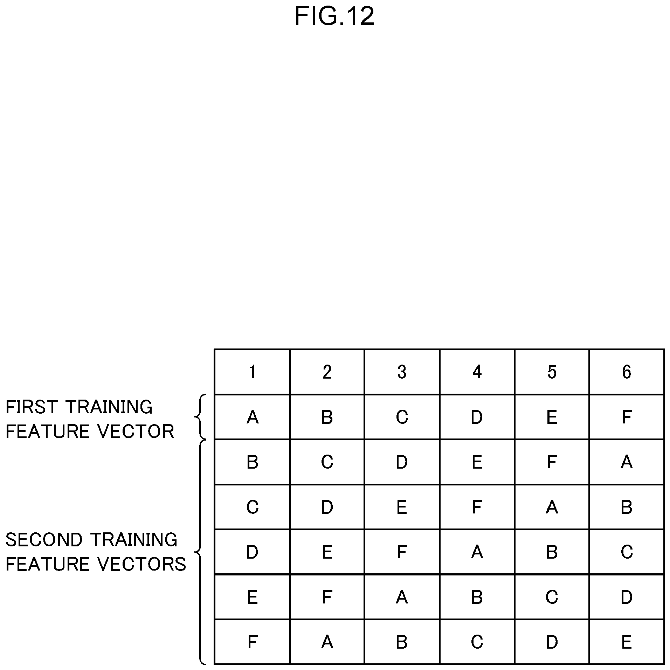

Fig. 12 is a diagram for explaining the generation of second training feature vectors. The number 1 and so on in Fig. 12 indicates positions of elements of the feature vectors. In the example illustrated in Fig. 12, a feature value B may be extracted from a first residue of a given cyclic peptide, a feature value C may extracted from a second residue, a feature value D may extracted from a third residue, and a feature value E may be extracted from a fourth residue. In order to convert a feature vector with a length of four into a feature vector with a length of six, a feature value A is inserted at the location of the number 1, and a feature value F is inserted at the location of the number 6. In this manner, the elements A, B, C, D, E, F become elements of the first training feature vector.

Next, as illustrated in Fig. 12, the training data generation section 315 cyclically shifts the elements A, B, C, D, E, F of the first training feature vector to the left by a distance of one, so as to generate a second training feature vector with the elements B, C, D, E, F, A. Then, in a similar manner, the elements A, B, C, D, E, F of the first training feature vector are cyclically shifted to the left by a distance of two, so as to generate a second training feature vector with the elements C, D, E, F, A, B. During this processing, positions in a sequence are shifted by a fixed distance without changing the order in the sequence between before and after, in a similar manner to rotation processing of a text string or a bit string, in processing that implements a wraparound at an end point. This processing obtains a first training feature vector and plural second training feature vectors from a single cyclic peptide, which can then be employed as training data.

The training data generation section 315 generates training data expressed by the first training feature vector set and the second training feature vectors set paired with respective correct values for membrane permeability of the training cyclic peptides. The training data generation section 315 then stores the plural generated items of training data in the training data storage section 16.

The training section 18 generates a trained model, for outputting a predicted value for membrane permeability of a cyclic peptide from feature vectors expressing cyclic peptide features, by executing a machine learning algorithm based on the plural training data stored in the training data storage section 16.

Note that other configuration and operation of the prediction device 310 of the third exemplary embodiment are similar to those of the first exemplary embodiment or second exemplary embodiment, and so explanation thereof is omitted.

As described above, the prediction device of the third exemplary embodiment extracts the first training feature vectors expressing features from the plural training cyclic peptides. For each of the first training feature vectors, the prediction device adjusts the length of the first training feature vector to a prescribed length, then cyclically shifts the elements of the first training feature vector so as to generate a set of second training feature vectors. The prediction device generates training data expressed by the first training feature vector set and the second training feature vectors set paired with correct values for membrane permeability of the respective training cyclic peptides. The prediction device then generates a trained model, for outputting a predicted value for membrane permeability of a cyclic peptide from feature vectors expressing features of a cyclic peptide, by executing a machine learning algorithm based on the plural generated items of training data. This enables the training data to be augmented while considering structural characteristic of the cyclic peptides. Moreover, the trained model can be obtained based on a large amount of training data generated in consideration of the configuration of the cyclic peptides.

Fourth Exemplary Embodiment

Next, explanation follows regarding a fourth exemplary embodiment. A prediction device of the fourth exemplary embodiment differs from the first to third exemplary embodiments in respect that a predicted value for membrane permeability of a cyclic peptide is generated using a convolutional neural network model including a layer in which elements at both ends of a feature vector are placed adjacent to one another so as to correspond to the structural properties of cyclic peptides. Note that similar portions in the configuration of the prediction device according to the fourth exemplary embodiment to any of those of the prediction devices of the first to third exemplary embodiments are allocated the same reference numerals, and explanation thereof is omitted.

There is a need for feature vectors extracted from a cyclic peptide to be expressed as a ring of the residues configuring the cyclic peptide. In this regard, vectors that are elements simply arrayed in a one-dimensional form result in a start end and a terminal end being created as a result, and thus might not be considered as appropriately expressing the continuity of the ring of residues in a cyclic peptide.

Thus, the prediction device of the fourth exemplary embodiment generates predicted values for membrane permeability of cyclic peptides using a convolutional neural network model including a both-end-adjacency layer in which elements at both ends of a feature vector are placed adjacent to one another. The configuration of the residues of the cyclic peptides are thereby expressed in the convolutional neural network model.

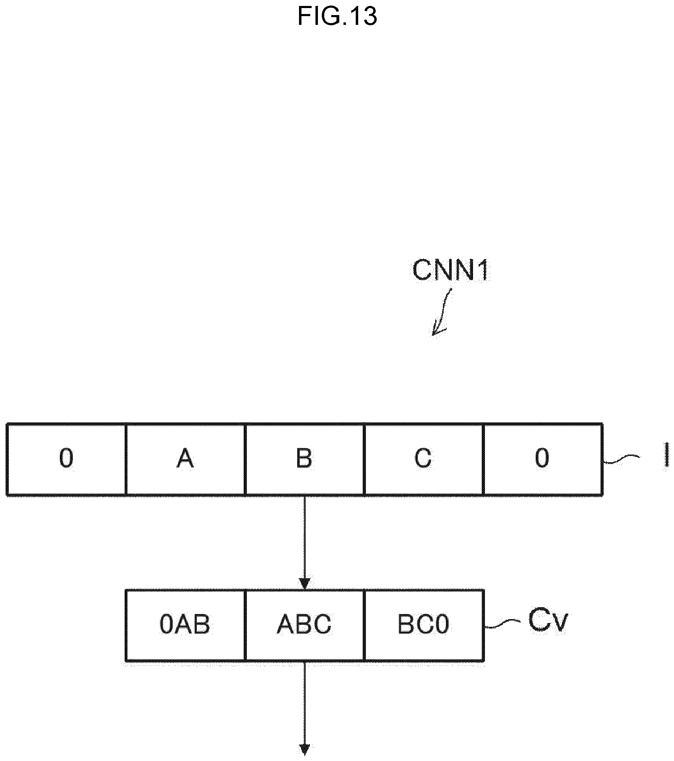

Fig. 13 is a configuration diagram of a conventional convolutional neural network model. As illustrated in Fig. 13, a conventional convolutional neural network model CNN1 includes an input layer I and a convolutional layer Cv. Note that illustration of other convolutional layers, pooling layers, and so on is omitted. As illustrated in Fig. 13, when a feature vector [0, A, B, C, 0] is input to the input layer I, convolutional processing is performed in the convolutional layer Cv such that [0, A, B], [A, B, C], and [B, C, 0] are extracted from the feature vector. However, in this conventional convolutional neural network model CNN1, convolutional processing is merely performed on the input feature vector, and no consideration is made of the structure of the cyclic peptide from which the feature vector was extracted.

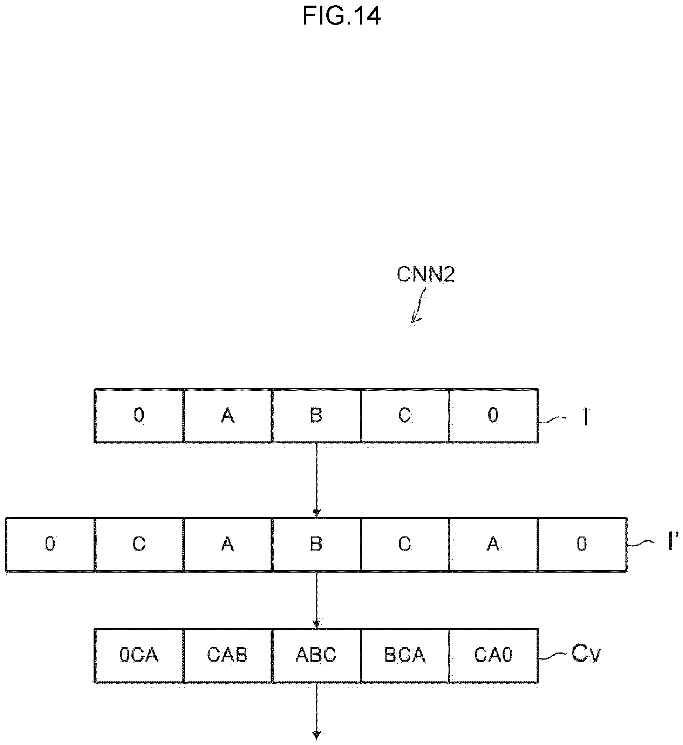

In contrast thereto a convolutional neural network model of the fourth exemplary embodiment includes a layer that considers the structural features of the cyclic peptide. Fig. 14 is a configuration diagram of the convolutional neural network model of the fourth exemplary embodiment. As illustrated in Fig. 14, a convolutional neural network model CNN2 of the fourth exemplary embodiment includes an input layer I, a convolutional layer Cv, and a both-end-adjacency layer I'. The both-end-adjacency layer I' is a layer in which elements at both ends of the feature vector are redisposed so as to be adjacent to each other on the left and right. Specifically, as illustrated in Fig. 14, C is disposed adjacent to the left side of A, and A is disposed adjacent to the right side of C. The ring of residues of the cyclic peptide is expressed in this manner.

Based on plural training data, the training section 18 of the fourth exemplary embodiment generates a trained convolutional neural network model, for outputting a predicted value for membrane permeability of a cyclic peptide from feature vectors, by training a convolutional neural network model including a both-end-adjacency layer in which elements at both ends of a training feature vector are placed adjacent to one another. The training section 18 then stores the trained convolutional neural network model in the trained model storage section 20.

The generation section 24 of the fourth exemplary embodiment generates predicted values for membrane permeability of prediction target peptides by inputting feature vectors extracted from a cyclic peptide that is the target for membrane permeability prediction into the trained convolutional neural network model stored in the trained model storage section 20.

Note that other configuration and operation of the prediction device 410 of the fourth exemplary embodiment are similar to those of a prediction device of the first to third exemplary embodiments, and so explanation thereof is omitted.

As described above, based on plural training data the prediction device of the fourth exemplary embodiment generates a trained convolutional neural network model for outputting a predicted value for membrane permeability of a cyclic peptide from feature vectors, by training a convolutional neural network model including a both-end-adjacency layer in which elements at both ends of training feature vectors are placed adjacent to one another. This enables a trained convolutional neural network model to be obtained that considers structural characteristic of cyclic peptides.

Moreover, the prediction device generates predicted values for membrane permeability of prediction target peptides by inputting feature vectors extracted from a cyclic peptide that is the target for membrane permeability prediction into the trained convolutional neural network model including the both-end-adjacency layer in which elements at both ends of a feature vector are placed adjacent to one another. This enables predicted values for membrane permeability to be obtained that consider structural characteristic of cyclic peptides.

Fifth Exemplary Embodiment

Next, explanation follows regarding a fifth exemplary embodiment. A prediction device of the fifth exemplary embodiment differs from the first to fourth exemplary embodiments in respect that predicted values for peptide membrane permeability are generated by molecular dynamics simulation. Note that similar portions in the configuration of the prediction device according to the fifth exemplary embodiment to those of any of the prediction devices of the first to fourth exemplary embodiments are allocated the same reference numerals, and explanation thereof is omitted.

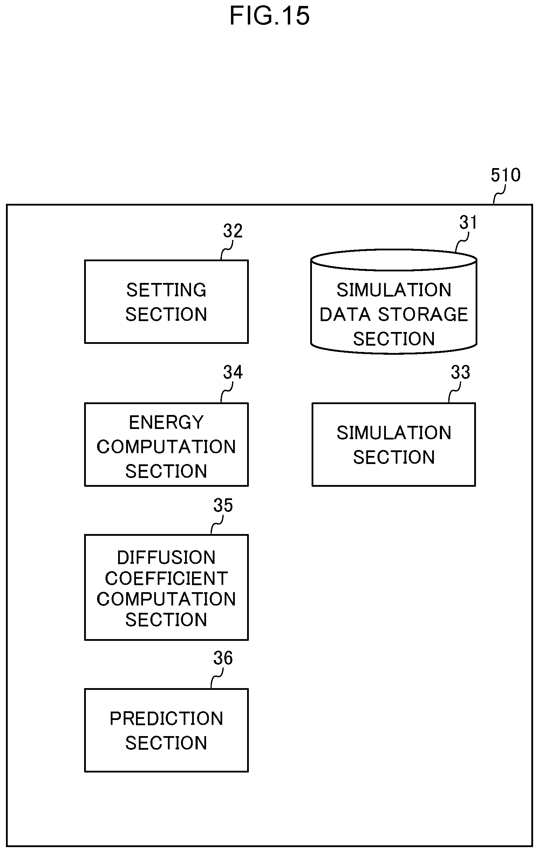

Fig. 15 is a block diagram illustrating an example of a configuration of a prediction device 510 according to the fifth exemplary embodiment. As illustrated in Fig. 15, in terms of functionality, the prediction device 510 includes a simulation data storage section 31, a setting section 32, a simulation section 33, an energy computation section 34, a diffusion coefficient computation section 35, and a prediction section 36.

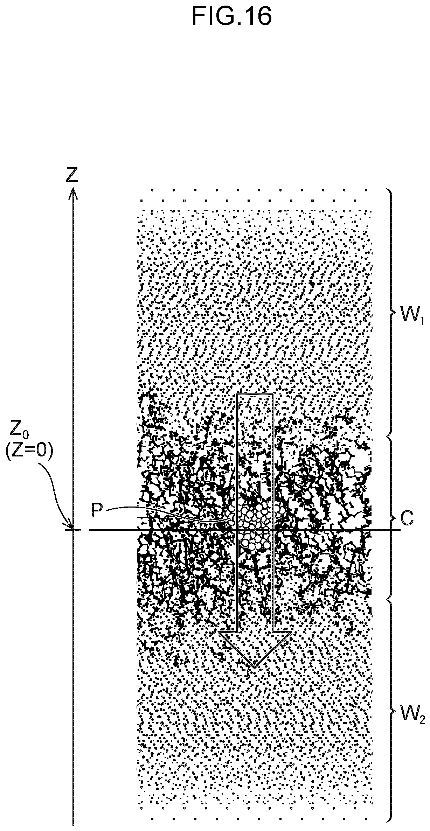

Fig. 16 is a diagram for explaining a manner in which peptides permeate a cell membrane. As illustrated in Fig. 16, a peptide P that has been virtually set in a computer is permeating a membrane region C representing a cell membrane. In such a case, the peptide P enters the membrane region C from a first solvent region $W_1$ representing a solvent adjacent to one side of the membrane region C, and permeates the membrane region C. The peptide P then arrives at a second solvent region $W_2$ representing a solvent adjacent to another side of the membrane region C. Note that a reaction coordinate z representing a position of the peptide P in a direction of an axis perpendicular to a membrane surface of the membrane region C penetrated by the peptide P is defined as illustrated in Fig. 16. The prediction device of the fifth exemplary embodiment simulates, by molecular dynamics simulation, the dynamics of the peptide permeating through the membrane region C, the first solvent region Wi, and the second solvent region $W_2$.

Various data for simulating pharmacokinetics of a peptide by molecular dynamics simulation is stored in the simulation data storage section 31. The simulation section 33, described later, simulates pharmacokinetics of the peptide based on the various data stored in the simulation data storage section 31. Note that data obtained from simulation is also stored in the simulation data storage section 31. The energy computation section 34, described later, computes free energy of the peptide based on the data obtained from simulation. The diffusion coefficient computation section 35, described later, computes a diffusion coefficient based on the data obtained from simulation. The prediction section 36 computes predicted values for membrane permeability based on the free energy of the peptide and the diffusion coefficient.

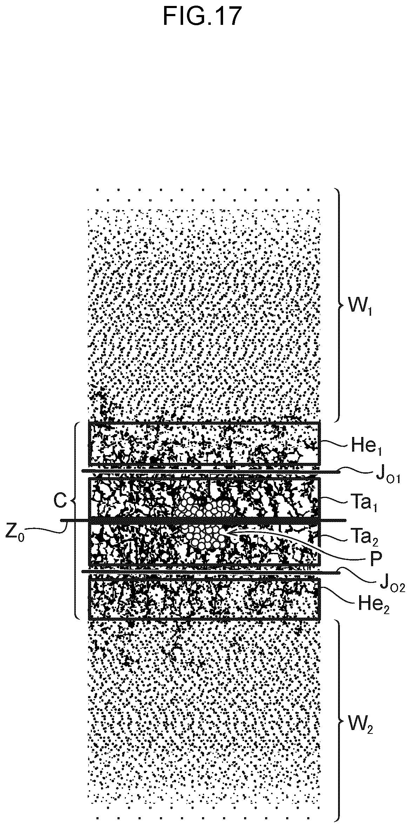

Fig. 17 is a diagram for explaining various locations in the molecular dynamics simulation of the fifth exemplary embodiment. As illustrated in Fig. 17, the first solvent region Wi, the membrane region C, and the second solvent region $W_2$ are present therein.

A tail Tai, a head $He_1$, and a lipid molecule join $Jo_1$ therebetween are present inside the membrane region C on the side adjacent to the first solvent region $W_1$. The join $Jo_1$ is positioned further toward a membrane center side than a boundary between the first solvent region $W_1$ and the membrane region C.

A tail $Ta_2$, a head $He_2$, and a join $Jo_2$ therebetween are present inside the membrane region C on the side adjacent to the second solvent region $W_2$. A membrane central zone Zo is present in a central area of the membrane region C.

Note that the membrane region C is a region spanning from the head $He_1$ in contact with the first solvent region $W_1$ across to the head $He_2$ in contact with the second solvent region $W_2$.

As can be seen from Fig. 17, there is a symmetrical relationship between a segment spanning from the first solvent region $W_1$ to the membrane central zone Zo that is the central area of the membrane region C, and a segment spanning from the membrane central zone Zo to the second solvent region $W_2$. Thus, by executing a molecular dynamics simulation from the first solvent region $W_1$ through to past the membrane central zone Zo, it is possible by utilizing this result to obtain a simulation result spanning from the membrane central zone Zo to the second solvent region $W_2$. This enables a reduction to be achieved in the time required for computation.

Thus, the following explanation describes a case in which a molecular dynamics simulation is executed for a segment spanning from the first solvent region $W_1$ to sufficiently past the membrane central zone Zo, and in which the simulation result thereof is utilized to obtain a simulation result for from the membrane central zone Zo to the second solvent region $W_2$.

The setting section 32 sets, as setting information when simulating permeation of the peptide P in the segment spanning from the first solvent region $W_1$ to as far as the vicinity of the join $Jo_1$, an initial conformation of the peptide P as an initial conformation corresponding to the relative substance permittivity in the first solvent region $W_1$.

The setting section 32 also sets, as setting information when simulating permeation of the peptide P in a segment spanning from the vicinity of the join $Jo_1$ to sufficiently past the membrane central zone Zo, an initial conformation of the peptide P as an initial conformation corresponding to the relative substance permittivity in the membrane region C. The initial conformation of the peptide is thereby set to correspond to the surrounding environment in which the peptide is present, enabling simulation of pharmacokinetics of the peptide.

The simulation section 33 executes a molecular dynamics simulation of pharmacokinetics of the peptide. For example, the simulation section 33 executes a molecular dynamics simulation of pharmacokinetics of the peptide using known simulation software such as AMBER (Internet URL = https://ambermd.org/ accessed February 8th 2021), or GROMACS (Internet URL = http://www.gromacs.org/ accessed February 8th 2021).

First, the simulation section 33 simulates permeation of the peptide P in the segment spanning from the first solvent region $W_1$ to as far as the vicinity of the join $Jo_1$, and also simulates permeation of the peptide P in the segment spanning from the vicinity of the join $Jo_1$ to a region sufficiently past the membrane central zone Zo, these corresponding to the initial conformation of the peptide as set by the setting section 32, and stores these simulation results in the simulation data storage section 31.

The setting section 32 acquires an initial conformation of simulation by replica exchange umbrella sampling (hereafter simply referred to as REUS simulation) executed subsequently to storing the simulation results in the simulation data storage section 31. The initial conformation is a series of initial conformations of the peptide in respective regions at the periphery of the membrane when simulating peptide dynamics by REUS simulation. The setting section 32 thereby sets the series of initial conformations in the REUS simulation based on the peptide dynamics simulation results.

Note that in the fifth exemplary embodiment the pharmacokinetics of the peptide are simulated by a known REUS simulation, as described later. When this is performed, there is a need to preset restraint positions of peptide replicas in the respective regions of the first solvent region $W_1$ and of the membrane region C.

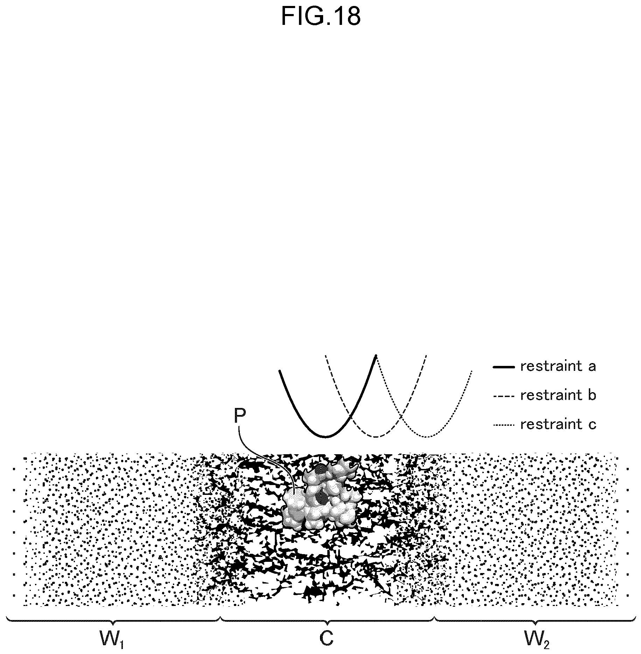

Fig. 18 is a diagram for explaining restraint positions of the peptide in the REUS simulation. As illustrated in Fig. 18, restraint positions ("restraint a", "restraint b", and "restraint c" in Fig. 18) that are reference points for restraint energy are set in the respective regions of the first solvent region $W_1$ and the membrane region C.

The setting section 32 sets such that a spacing between the restraint positions of the peptide replicas is finer (or narrower) closer a region is to the membrane central zone Zo at the central area of the membrane region C. This enables a smooth exchange of replicas to be performed in the central portion of the membrane where the change in free energy is predicted to be large. As a result this enables efficient sampling of conformation and the like of the peptide inside the membrane.

In a REUS simulation, restraint positions of replicas are set slightly offset from one another, and the structure etc. of the simulation target is exchanged between the respective restraint positions. In the fifth exemplary embodiment also, the replica restraint positions are set slightly offset from one another, and conformation of the peptide is exchanged between the respective restraint positions. A peptide with a new conformation is therefore anticipated to be found at each of the restraint positions.

There is a steep change in free energy of the peptide in the vicinity of the membrane central zone Zo at the central area of the membrane region C due to different reaction coordinates z. Namely, the membrane central zone $Z_0$ is considered to be a so-called difficult region when the peptide permeates the cell membrane. To address this, in the fifth exemplary embodiment, the replica restraint positions of the peptide are set at a narrower spacing the closer to the membrane central zone Zo at the central area of the membrane region C, such that more information is reflected in the simulation that relates to conformation of the peptide in the vicinity of the membrane central zone Zo.

In currents computers, the simulation of cell membrane permeability of a peptide is a simulation having an extremely high computation cost. The greater the number of peptide replicas, the higher the computation cost of the simulation. Thus, rather than setting the spacing of replica restraint positions simply at a uniform spacing, setting the restraint positions at a narrower spacing the closer to the membrane central zone Zo, this being the region with greatest impact on membrane permeability prediction results, as in the fifth exemplary embodiment, enables simulation results to be obtained at good accuracy while suppressing computation cost.

The simulation section 33 then executes a REUS simulation based on the replica restraint positions set by the setting section 32 and on a series of initial conformations of the peptide in the respective regions in the vicinity of the cell membrane, and stores the result thereof in the simulation data storage section 31.

The energy computation section 34 computes a free energy G(z) of the peptide at the respective reaction coordinates z according to a known computation formula based on the REUS simulation result stored in the simulation data storage section 31. Specifically, the energy computation section 34 computes the free energy G(z) of the peptide based on the information regarding relative position coordinates of the membrane and the peptide in the REUS simulation result stored in the simulation data storage section 31.

Next, the energy computation section 34 computes for each of the reaction coordinates z when the REUS simulation has executed a difference $\Delta G(z)$ between a minimum value $G_{min}$ out of the free energies G(z) of the peptide computed for each of the reaction coordinates z and the free energy G(z) of the peptide at the reaction coordinate z.

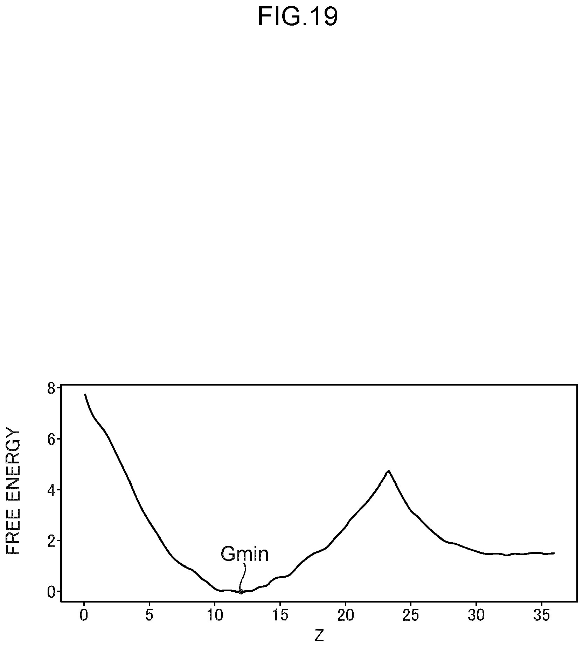

Fig. 19 is a diagram for explaining the minimum value $G_{min}$ out of the free energies G(z) of the peptide. As illustrated in Fig. 19, the free energy G(z) is computed at the respective reaction coordinates z. In this case, the minimum value $G_{min}$ out of the free energies G(z) at the respective reaction coordinates z is the value illustrated in Fig. 19.

Next, the simulation section 33 executes a simulation using an umbrella sampling method (hereafter simply referred to as US simulation), and stores the simulation result in the simulation data storage section 31. Specifically, the simulation section 33 obtains the US simulation result by performing umbrella sampling using a final structure (such as a series of initial conformations) in the results of REUS simulation stored in the simulation data storage section 31. Note that exchange of replicas is not implemented when this umbrella sampling is performed. The US simulation result includes, for each of the reaction coordinates z, a value var(z) expressing variance in peptide centroid position, and a value Czz(t) expressing autocorrelation of centroid position at respective timings t.

Note that the simulation section 33 takes an inverse of the simulation result for the segment spanning from the first solvent region $W_1$ as far as the membrane central zone Zo in the simulation data storage section 31 and stores this as a simulation result for the segment spanning from the membrane central zone Zo as far as the second solvent region $W_2$.

The diffusion coefficient computation section 35 computes a local diffusion coefficient D(z) at each of the reaction coordinates z based on the US simulation result stored in the simulation data storage section 31. Specifically, the diffusion coefficient computation section 35 computes at each of the reaction coordinates z the diffusion coefficient D(z) according to Equation (2) below based on the value var(z) expressing variance in peptide centroid position, and the value Czz(t) expressing autocorrelation in centroid position at respective timings t.

$$D(z) = \frac{\text{var}(z)^2}{\int_0^\infty C_{zz}(t)\, dt}$$

$$(2)$$

The prediction section 36 computes at each of the respective reaction coordinates z a value R(z) expressing local resistance at the reaction coordinate z of the peptide according to Equation (3) below and based on the difference $\Delta G(z)$ and the local diffusion coefficient D(z) at the reaction coordinate z. Note that $\beta$ in the following Equation is a preset coefficient.

$$R(z) = \frac{\exp(\beta \Delta G(z))}{D(z)}$$

(3)

The prediction section 36 then computes a predicted value $P_{eff}$ for membrane permeability of the peptide according to Equation (4) below and based on the value R(z) expressing the local resistance computed at the respective reaction coordinates z.

$$P_{eff} = \frac{1}{R_{eff}} = \frac{1}{\int_{z_a}^{z_b} R(z)dz}$$

(4)

Note that $z_a$, $z_b$ in the above Equation are coordinates expressing ends of the reaction coordinates z in the simulation. Note that in cases in which a conventional method is adopted (see for example Siewert-Jan Marrink and Herman J. C. Berendsen, "Simulation of water transport through a lipid membrane", J. Phys. Chem. 1994, 98, 15, 4155-4168), $z_a$ is set as the first solvent region Wi, and $z_b$ is set as the second solvent region $W_2$.

The prediction section 36 of the present exemplary embodiment sets as a reaction coordinate z at a position $z_a$ corresponding the minimum value $G_{min}$ out of the free energies G(z). The prediction section 36 then sets $z_b$ as the membrane central zone Zo, and computes membrane permeability $P_{flip}$ by computing the right side of Equation (4). Moreover, the prediction section 36 sets $z_b$ as the first solvent region $W_1$ and computes membrane permeability $P_{out}$ by computing the right side of Equation (4).

The prediction section 36 then takes the lower value out of the membrane permeability $P_{flip}$ and the membrane permeability $P_{out}$ to predict a predicted value $P_{eff}$ for membrane permeability.

Note that the above simulations may, for example, be executed based on the following Reference Cited Document.

Reference Cited Document 1: Yuji Sugita, Akio Kitao, and Yuko Okamoto, "Multidimensional replica-exchange method for free-energy calculations", J. Chem. Phys. 2000, 113, 15, 6042-6051.

Next, explanation follows regarding operation of the prediction device 510 of the fifth exemplary embodiment.

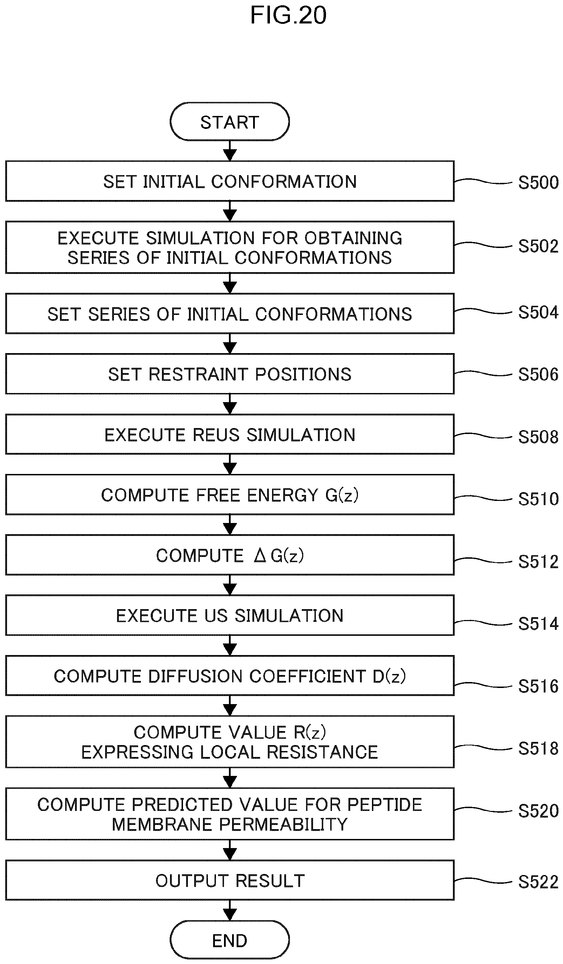

On receiving an instruction signal indicating an instruction to start simulation, the prediction device 510 of the fifth exemplary embodiment executes the simulation processing routine illustrated in Fig. 20.

At step S500, the setting section 32 sets an initial conformation of the peptide P when simulating permeation of the peptide P in a segment spanning from the first solvent region $W_1$ to the vicinity of the join $Jo_1$ as an initial conformation corresponding to relative substance permittivity in the first solvent region $W_1$. The setting section 32 also sets an initial conformation of the peptide P when simulating permeation of the peptide P in a segment spanning from the vicinity of the join $Jo_1$ to sufficiently past the membrane central zone Zo as an initial conformation corresponding to relative substance permittivity in the membrane region C.

At step S502, the simulation section 33 executes a simulation of the peptide P permeating the segment spanning from the first solvent region $W_1$ to the vicinity of the join $Jo_1$, and executes a simulation of the peptide P permeating the segment spanning from the vicinity of the join $Jo_1$ to sufficiently past the membrane central zone Zo according to the peptide initial conformations set at step S500. The simulation section 33 then stores the simulation results in the simulation data storage section 31. Note that these simulation results include the series of initial conformations of the peptide in the respective regions.

At step S504, the setting section 32 sets the series of initial conformations obtained at step S502 as the initial conformations to be employed in the REUS simulation, described later.

At step S506, the setting section 32 sets the spacing between restraint positions of the peptide replicas when executing the REUS simulation such that the spacing between the restraint positions of the peptide replicas become finer the closer a region is to the membrane central zone Zo.

At step S508, the simulation section 33 executes simulation of pharmacokinetics of the peptide by REUS simulation based on the series of initial conformations set at step S504 and on the replica restraint positions set at step S506. The simulation section 33 then stores the REUS simulation results in the simulation data storage section 31.

At step S510, the energy computation section 34 computes the free energy G(z) of the peptide at the respective reaction coordinates z according to a known computation formula and based on the REUS simulation result stored in the simulation data storage section 31.

At step S512, for each of the reaction coordinates z, the energy computation section 34 computes the difference $\Delta G(z)$ between the minimum value $G_{min}$ out of the free energies G(z) of the peptide computed at the reaction coordinates z, and the free energy G(z) of the peptide at the reaction coordinate z, based on the results of REUS simulation stored in the simulation data storage section 31.

At step S514, the simulation section 33 executes a US simulation based on a series of end structures, these being the results of the simulation performed at step S508.

Next, at step S516, the diffusion coefficient computation section 35 computes the local diffusion coefficient D(z) based on the value var(z) expressing variance of centroid position of the peptide when executing the US simulation for the respective reaction coordinates z in the results of the simulation executed at step S514, and based on the value Czz(t) expressing autocorrelation in centroid position at respective timings t.

At step S518, the prediction section 36 computes, for each of the reaction coordinates z in the results of the simulation executed at step S514, the value R(z) expressing local resistance of the peptide at the reaction coordinate z based on the difference $\Delta G(z)$ and the local diffusion coefficient D(z) at the reaction coordinate z.

At step S520, the prediction section 36 computes a predicted value for membrane permeability of the peptide based on the value R(z) expressing the local resistance computed at the respective reaction coordinates z.

At step S522, the prediction section 36 outputs as a result the predicted value for peptide membrane permeability computed at step S520.

As described above, the prediction device of the fifth exemplary embodiment computes a predicted value for membrane permeability of a peptide when permeating a membrane region representing a cell membrane, a first solvent region representing a solvent adjacent to one side of the membrane region, and a second solvent region representing a solvent adjacent to the other side of the membrane region. The prediction device computes the free energy G(z) of the peptide at respective reaction coordinates z expressing positions of the peptide in regions including the first solvent region, the membrane region, and the second solvent region, and also expressing the position of the peptide in a direction of an axis perpendicular to the membrane surface of the membrane region. The prediction device computes at each of the respective reaction coordinates z the difference $\Delta G(z)$ between the minimum value $G_{min}$ out of the free energies G(z) of the peptide computed at the reaction coordinates z and the free energy G(z) of the peptide at the reaction coordinate z. The prediction device also computes, for each of the respective reaction coordinates z, the value R(z) expressing local resistance of the peptide at the reaction coordinate z based on the difference $\Delta G(z)$ and based on the local diffusion coefficient D(z) at the reaction coordinate z. The prediction device then computes a predicted value for membrane permeability of the peptide based on the value R(z) expressing the local resistance computed at the respective reaction coordinates z. This enables the membrane permeability of the peptide to be predicted at good accuracy. In conventional methods, $\Delta G(z)$ at the respective reaction coordinates z has been computed based on an energy reference value outside the cell membrane. In contrast thereto, the prediction device of the fifth exemplary embodiment uses the minimum value $G_{min}$ out of the free energy of the peptide inside the cell membrane to compute $\Delta G(z)$ at the respective reaction coordinates z, thereby enabling the dynamics of the peptide inside the cell membrane to be simulated at good accuracy, and the membrane permeability of the peptide to be predicted at good accuracy.

Specifically, as is evident from Fig. 19, dynamics are envisaged in which, after the peptide has penetrated the membrane region C and reached the state corresponding to the minimum value $G_{min}$ out of the free energy, the peptide then passes the membrane central zone, i.e. the difficult region, and permeates the membrane region. However, in conventional methods, a change $\Delta G(z)$ in the free energy of the peptide has been computed using a free energy value outside the membrane region C as a reference value, and the size of the barrier when the peptide permeates from a stable state through the difficult region has not been appropriately linked to membrane permeability. In contrast thereto, in the prediction device of the fifth exemplary embodiment, the change $\Delta G(z)$ in free energy is computed using the minimum value $G_{min}$ out of the free energies G(z) of the peptide, thereby enabling the membrane permeability of the peptide to be predicted at good accuracy.

Moreover, the prediction device of the fifth exemplary embodiment sets an initial conformation of the peptide corresponding to relative substance permittivity in the first solvent region when simulating permeation of the peptide in a segment spanning from the first solvent region to the vicinity of a lipid molecule join positioned further toward the membrane center side than a boundary between the first solvent region and the membrane region. The prediction device also sets an initial conformation of the peptide corresponding to relative substance permittivity in the membrane region when simulating permeation of the peptide in a segment spanning from the vicinity of the join to sufficiently past the membrane central zone expressing the central area of the membrane region. The prediction device then predicts membrane permeability of the peptide by simulating dynamics of the peptide corresponding to the initial conformations of the peptide that were set. This enables the initial conformations of the peptide to be set as initial conformations corresponding to relative substance permittivity in the membrane region. As a result this enables the dynamics of the peptide inside the cell membrane to be simulated at good accuracy, enabling the membrane permeability of the peptide to be predicted at good accuracy.

Moreover, the prediction device of the fifth exemplary embodiment performs setting such that spacing between the restraint positions of the peptide are finer the closer a region is to the membrane central zone expressing the central area of the membrane region when simulating peptide permeation using a replica exchange umbrella sampling method. The prediction device then predicts the membrane permeability of the peptide by simulating dynamics of the peptide using the replica exchange umbrella sampling method according to the spacing between restraint positions that were set. Setting the restraint positions at a finer spacing the closer to the membrane central zone, this being the region with greatest impact on the prediction results for membrane permeability of the peptide, enables simulation results to be obtained with good accuracy while suppressing computation cost, thereby enabling the membrane permeability of the peptide to be predicted at good accuracy.

Sixth Exemplary Embodiment

Next, explanation follows regarding a sixth exemplary embodiment. A prediction device of the sixth exemplary embodiment differs from the first to fifth exemplary embodiments in respect that a predicted value for peptide membrane permeability is computed by consolidating a predicted value for peptide membrane permeability obtained by molecular dynamics simulation with a predicted value for membrane permeability obtained by a trained model built by machine learning. Note that similar portions in the configuration of the prediction device according to the sixth exemplary embodiment to those of any of the prediction devices of the first to fifth exemplary embodiments are allocated the same reference numerals, and explanation thereof is omitted.

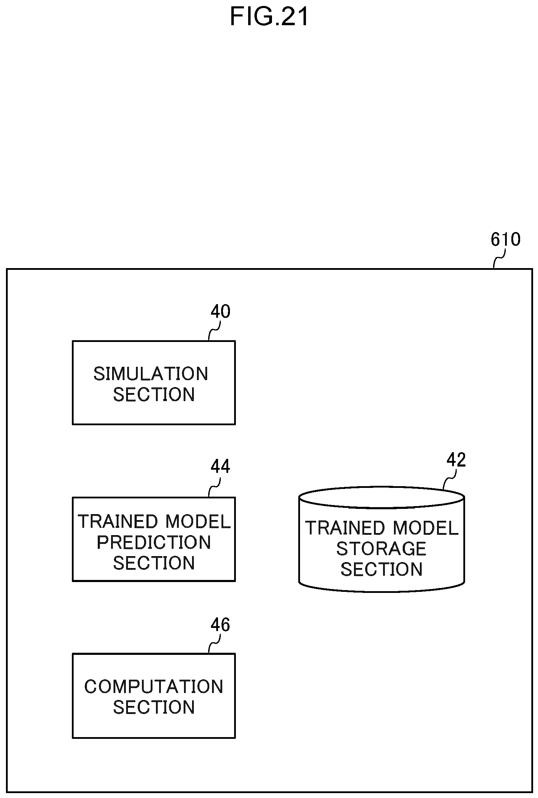

Fig. 21 is a block diagram illustrating an example of a configuration of a prediction device 610 according to the sixth exemplary embodiment. As illustrated in Fig. 21, in terms of functionality, the prediction device 610 includes a simulation section 40, a trained model storage section 42, a trained model prediction section 44, and a computation section 46.

The simulation section 40 generates a first membrane permeability value expressing membrane permeability of a peptide by simulating dynamics of the peptide permeating through a membrane region representing a cell membrane, a first solvent region representing a solvent adjacent to one side of the membrane region, and a second solvent region representing a solvent adjacent to the other side of the membrane region. For example, the simulation section 40 generates the first membrane permeability value expressing membrane permeability of the peptide by a similar method to that of the prediction device of the fifth exemplary embodiment.

A trained model for outputting a predicted value for membrane permeability of a peptide from feature vectors is stored in the trained model storage section 42. For example, a trained model generated using any one of the prediction devices of the first to fourth exemplary embodiments is stored in the trained model storage section 42.

The trained model prediction section 44 extracts a predictive feature vector expressing a feature from the peptide that is the target for membrane permeability prediction, and generates a second membrane permeability value expressing membrane permeability of the peptide by inputting these predictive feature vectors into the trained model stored in the trained model storage section 42.

The computation section 46 computes a predicted value for membrane permeability of the peptide by consolidating the first membrane permeability value generated by the simulation section 40 with the second membrane permeability value generated by the trained model prediction section 44. For example, the computation section 46 may compute a predicted value for membrane permeability of the peptide by averaging the first membrane permeability value and the second membrane permeability value. Alternatively, the computation section 46 may compute the larger or smaller value out of the first membrane permeability value or the second membrane permeability value as being the predicted value for membrane permeability of the peptide.

The computation section 46 outputs this predicted value for membrane permeability of the peptide as a result.

As described above, the prediction device of the sixth exemplary embodiment generates the first membrane permeability value expressing peptide membrane permeability by simulating dynamics of the peptide. The prediction device also extracts predictive feature vectors expressing features from the peptide, and generates the second membrane permeability value expressing peptide membrane permeability by inputting the predictive feature vectors into a pre-built trained model. The prediction device then computes a predicted value for membrane permeability of the peptide by consolidating the generated first membrane permeability value with the generated second membrane permeability value. This enables a predicted value to be obtained that reflects both a predicted value obtained by molecular dynamics simulation and a predicted value obtained using a trained model.

Seventh Exemplary Embodiment

Next, explanation follows regarding a seventh exemplary embodiment. A prediction device of the seventh exemplary embodiment differs from the first to sixth exemplary embodiments in respect that a trained model that employs a machine learning algorithm is built based on data of simulation results obtained by a molecular dynamics simulation. Note that similar portions in the configuration of the prediction device according to the seventh exemplary embodiment to any of those in the prediction devices of the first to sixth exemplary embodiments are allocated the same reference numerals, and explanation thereof is omitted.

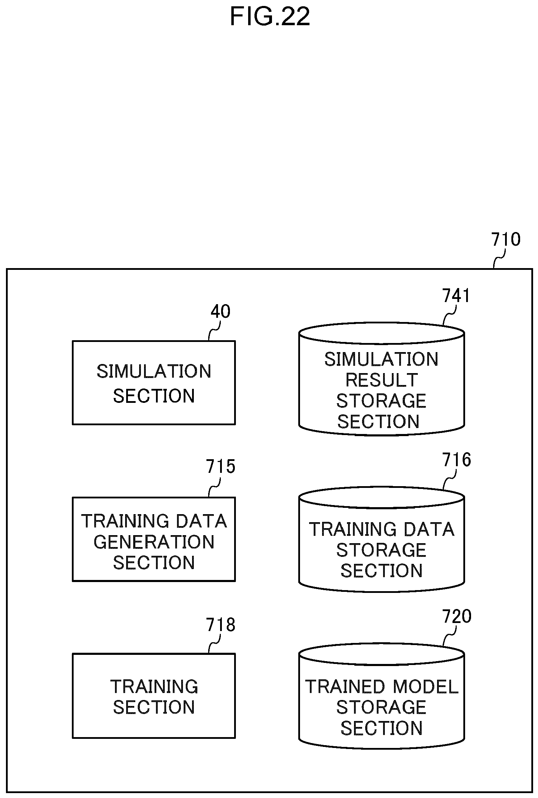

Fig. 22 is a block diagram illustrating an example of a configuration of a prediction device 710 according to the seventh exemplary embodiment. As illustrated in Fig. 22, in terms of functionality, the prediction device 710 includes the simulation section 40, a simulation result storage section 741, a training data generation section 715, a training data storage section 716, a training section 718, and a trained model storage section 720.

The simulation section 40 simulates dynamics of a peptide permeating a membrane region C, a first solvent region W1, and a second solvent region $W_2$, similarly to in the sixth exemplary embodiment. The simulation section 40 then stores the simulation result obtained by this simulation in the simulation result storage section 741. Note that the simulation result includes a predicted value for peptide membrane permeability obtained by the simulation section 40, physical quantities in respective region locations, tertiary structure of the peptide in the respective region locations, and so on.

The simulation result obtained by the simulation section 40 is stored in the simulation result storage section 741.

The training data generation section 715 generates simulation-derived training data expressed by the predicted value for peptide membrane permeability stored in the simulation result storage section 741 paired with a feature vector generated from a 3D descriptor obtained from the tertiary structure of the peptide in the respective region locations. Note that the respective region locations correspond to positions corresponding to several representative reaction coordinates z in the first solvent region Wi, the membrane region C, and the second solvent region $W_2$.

Specifically, the training data generation section 715 obtains 3D descriptors at the respective locations in the tertiary structure of the peptide in the respective region locations included in the simulation result, and extracts a single or plural feature vectors from these 3D descriptors. The training data generation section 715 generates as the simulation-derived training data the extracted feature vector set paired with the predicted values for peptide membrane permeability included in the simulation results, and stores this in the training data storage section 716.

Plural items of simulation-derived training data are stored in the training data storage section 716.

The training section 718 generates a trained model, for outputting a predicted value for membrane permeability from feature vectors expressing features of a peptide and also expressing tertiary structure of the peptide, by executing a machine learning algorithm based on training data including the simulation-derived training data stored in the training data storage section 716. The training section 718 then stores the trained model in the trained model storage section 720. Note that the training data storage section 716 may also contain training data other than the simulation-derived training data.

Moreover, instead of predicted values for peptide membrane permeability, the training data may be configured by a physical quantity computed from the peptide and its surrounding environment at respective locations when executing a simulation, combined with feature vectors extracted from the 3D descriptors. In such cases, a trained model for predicting a physical quantity of a peptide from feature vectors is generated as result.

As described above, the prediction device of the seventh exemplary embodiment generates a predicted value for membrane permeability expressing membrane permeability of a peptide by simulating the dynamics of the peptide. The prediction device also generates simulation-derived training data expressed by a predicted value for peptide membrane permeability or a physical quantity computed from the peptide and its surrounding environment at respective locations, paired with a feature vector generated from a 3D descriptor obtained from a tertiary structure of the peptide at the respective locations. The prediction device then generates a trained model by executing a machine learning algorithm based on training data including the simulation-derived training data. This enables a trained model to be obtained for outputting a predicted value for membrane permeability from feature vectors based on data obtained by molecular dynamics simulation.

Note that the present disclosure is not limited to the exemplary embodiments described above, and various modifications and applications may be implemented within a range not departing from the spirit of the present disclosure.

For example, although an example has been described in the first exemplary embodiment of a case in which each feature vector is extracted for instances in which each of the plural residues contained in a cyclic peptide are at the start point of the cyclic sequence, these plural feature vectors are input into a trained model, and a representative value is obtained for the predicted values of membrane permeability output from the trained model, there is no limitation thereto. For example, a single feature vector may be generated from each of the feature vectors for instances in which each of the plural residues contained in the cyclic peptide are at the start point of the cyclic sequence, this single feature vector input into a trained model, so as to obtain a predicted value of membrane permeability. In such a case, for example, the single feature vector may be generated by taking a weighted average of the plural feature vectors. Moreover, for example, specific feature vectors may be selected from out of plural feature vectors, and a single feature vector generated by taking a weighted average of the plural feature vectors that have been selected. Moreover, even when generating the trained model, a single training feature vector may be generated from each of the training feature vectors for instances in which each of the plural residues contained in the cyclic peptide are at the start point of the cyclic sequence, and then this training feature vector employed so as to generate the trained model.

Moreover, although an example has been described of a case in which the simulation section 33 of the fifth exemplary embodiment described above executes REUS simulation based on the replica restraint positions set by the setting section 32 and on the series of initial conformations of peptide at the respective regions in the vicinity of the cell membrane, there is no limitation thereto. For example, instead of REUS simulation, a US simulation or a metadynamics simulation (Alessandro Laio and Michele Parrinello, "Escaping free-energy minima", Proc. Natl. Acad. Sci., 2002, 99, 12562-12566.) may be executed.

Note that although an example has been described of a case in the fifth exemplary embodiment described above in which the result of simulation for the segment from the first solvent region $W_1$ to the vicinity of the membrane central zone Zo is inverted to obtain the result of simulation from the membrane central zone Zo to the second solvent region $W_2$, there is no limitation thereto. The results of simulation from the membrane central zone Zo to the second solvent region $W_2$ may be obtained by executing an actual simulation from the membrane central zone Zo to the second solvent region $W_2$.

Moreover, although in the above exemplary embodiment examples have been described of cases in which the trained model is generated based on training data, there is no limitation thereto. For example, the trained model of the present exemplary embodiment may be generated as a distillation model based on other trained models.

Moreover, although embodiments have been described above in which a program according to the present disclosure is pre-stored (installed) in a storage section (not illustrated in the drawings), the program according to the present disclosure may be provided in a format recorded on a recording medium such as a CD-ROM, a DVD-ROM, a micro SD card, or the like.

Note that although in the above exemplary embodiments a CPU reads in software (a program) and executes processing thereof, various processors other than a CPU may be employed for execution. Processors in such cases include programmable logic devices (PLD) that allow circuit configuration to be modified post-manufacture, such as a field-programmable gate array (FPGA), and dedicated electric circuits, these being processors including a circuit configuration custom-designed to execute specific processing, such as an application specific integrated circuit (ASIC). The processing may be executed by any one of these various types of processor, or may be executed by a combination of two or more of the same type or different types of processor (such as plural FPGAs, or a combination of a CPU and an FPGA). The hardware structure of these various types of processors is more specifically an electric circuit combining circuit elements such as semiconductor elements.

Moreover, the respective processing of the exemplary embodiments may be executed by the processing being executed by a program in a configuration of a computer, a server, or the like including a generic computation processing device, a storage device, and the like. Such a program may be stored in a storage device or recorded on a recording medium such as a magnetic disc, an optical disc, or semiconductor memory, or provided over a network. Obviously, other configuration elements also do not need to be implemented using a single computer or server, and may be distributed across and implemented by plural computers that are connected together over a network.

The disclosures of Japanese Patent Application No. 2021-031234, filed on February 26, 2021, are incorporated herein by reference in their entirety. All publications, patent applications, and technical standards mentioned in this specification are herein incorporated by reference to the same extent as if each individual publication, patent application, or technical standard was specifically and individually indicated to be incorporated by reference.

**Claims**

1. A prediction device comprising:

   an extraction section configured to extract a predictive feature vector expressing a feature from a peptide that is a target for membrane permeability prediction;

an adjustment section configured to adjust a length of the predictive feature vector extracted by the extraction section to a prescribed length; and

a generation section configured to generate a predicted value of membrane permeability of the prediction target peptide by inputting the predictive feature vector, which has been adjusted in length by the adjustment section, into a trained model pre-trained to output a predicted value of peptide membrane permeability from a feature vector expressing a feature of a peptide.

2. The prediction device of claim 1, wherein the adjustment section is configured to adjust the length of the predictive feature vector by a padding method or by conversion using a linear interpolation method.

3. A trained model generation device comprising:

a training extraction section configured to extract a training feature vector expressing a feature from each of a plurality of training peptides;

a training adjustment section configured to adjust a length of each training feature vector for each of the plurality of training peptides extracted by the training extraction section to a prescribed length; and

a training section configured to generate a trained model, for outputting a predicted value of peptide membrane permeability from a feature vector expressing a feature of a peptide, by executing a machine learning algorithm based on training data that is the training feature vectors adjusted in length by the training adjustment section paired with correct values of membrane permeability of the training peptides.

4. A prediction device comprising:

an extraction section configured to extract each predictive feature vector expressing a feature from a cyclic peptide that is a target for membrane permeability prediction for cases in which each of a plurality of residues contained in the cyclic peptide is at a start point of a cyclic sequence; and

a generation section configured to generate a predicted value of membrane permeability of the prediction target cyclic peptide by inputting a plurality of the predictive feature vectors extracted by the extraction section into a trained model pre-trained to output a predicted value of peptide membrane permeability from a feature vector expressing a feature of a cyclic peptide.

5. The prediction device of claim 4, wherein the generation section is configured to input each of the plurality of predictive feature vectors into the trained model and to generate a representative value of a predicted value of membrane permeability of the prediction target cyclic peptide for each of a plurality of feature vectors output from the trained model.

6. A trained model generation device comprising:

a training extraction section configured to extract a training feature vector expressing a feature from out from among a plurality of training cyclic peptides for cases in which each of a plurality of residues contained in the respective training cyclic peptide is at a start point of a cyclic sequence; and

a training section configured to generate a trained model, for outputting a predicted value of membrane permeability of a cyclic peptide from a feature vector expressing a feature of a cyclic peptide, by executing a machine learning algorithm based on training data that is the plurality of training feature vectors extracted by the training extraction section for each of a plurality of training cyclic peptides paired with a correct value of membrane permeability of the respective training cyclic peptide.

7. A prediction device that comprises an energy computation section, a diffusion coefficient computation section, and a prediction section and that is configured to compute a predicted value of membrane permeability of a peptide permeating through a membrane region representing a cell membrane, a first solvent region representing a solvent adjacent to one side of the membrane region, and a second solvent region representing a solvent adjacent to another side of the membrane region, wherein:

the energy computation section is configured to, based on a result of simulation of a peptide permeating through the first solvent region, the membrane region, and the second solvent region, compute a free energy $G(z)$ of the peptide for each reaction coordinate $z$ expressing a position of the peptide in a region including the first solvent region, the membrane region, and the second solvent region by expressing the position of the peptide in a direction of an axis perpendicular to a membrane surface of the membrane region, and compute for each of the reaction coordinates z a difference $\Delta G(z)$ between a minimum value $G_{min}$ out from among the free energies $G(z)$ of the peptide computed for the respective reaction coordinates z and a free energy $G(z)$ of the peptide at the reaction coordinate z;

the diffusion coefficient computation section computes a local diffusion coefficient $D(z)$ for each of the reaction coordinates z; and

the prediction section computes a value $R(z)$ expressing a local resistance of the peptide at the reaction coordinate z based on the difference $\Delta G(z)$ computed by the energy computation section for the respective reaction coordinates z and based on the local diffusion coefficient $D(z)$ computed by the diffusion coefficient computation section, and computes a predicted value of membrane permeability of the peptide based on the value $R(z)$ expressing the local resistance computed for each of the reaction coordinates z.

8. The prediction device of claim 7, wherein the prediction section computes the local diffusion coefficient $D(z)$ based on a value $var(z)$ expressing a variance of position of a centroid of a peptide when executing umbrella sampling for each of the reaction coordinates z and based on a value $Czz(t)$ expressing an autocorrelation of the centroid positions at each time t.

9. A prediction device that comprises a setting section, a simulation section and a prediction section and is configured to simulate dynamics of a peptide permeating through a membrane region representing a cell membrane, a first solvent region representing a solvent adjacent to one side of the membrane region, and a second solvent region representing a solvent adjacent to another side of the membrane region, wherein:

the setting section is configured to:

set an initial conformation of the peptide according to a relative substance permittivity in the first solvent region for simulation of the peptide permeating a segment spanning from the first solvent region to a vicinity of a lipid molecule join positioned further toward a membrane center side than a boundary between the first solvent region and the membrane region,

set an initial conformation of the peptide according to a relative substance permittivity in the membrane region for simulation of the peptide permeating a segment spanning from the vicinity of the join to past a region of a membrane central zone representing a central area of the membrane region;

the simulation section is configured to simulate dynamics of the peptide according to the initial conformation of the peptide set by the setting section;

the setting section is configured to set a series of initial conformations at respective regions using an umbrella sampling method based on a result of simulation obtained by the simulation section;

the simulation section is configured to simulate the dynamics of the peptide according to an umbrella sampling method based on the series of initial conformations for each of the regions set by the setting section; and

the prediction section is configured to predict membrane permeability of the peptide based on a result of simulation based on an umbrella sampling method obtained by the simulation section.

10. A prediction device that comprises a setting section, a simulation section and a prediction section and is configured to simulate dynamics of a peptide permeating through a membrane region representing a cell membrane, a first solvent region representing a solvent adjacent to one side of the membrane region, and a second solvent region representing a solvent adjacent to another side of the membrane region, wherein:

when simulating permeation of the peptide using an umbrella sampling method, the setting section is configured to set a spacing between restraint positions of the peptide so as to be finer the closer a region is to a membrane central zone representing a central area of the membrane region;

the simulation section is configured to simulate dynamics of the peptide using an umbrella sampling method according to the spacing between the restraint positions set by the setting section; and

the prediction section is configured to predict membrane permeability of the peptide based on a result of the simulation obtained by the simulation section.

11. A prediction device comprising:

a simulation section configured to generate a first membrane permeability value expressing a membrane permeability of a peptide by simulating dynamics of the peptide permeating through a membrane region representing a cell membrane, a first solvent region representing a solvent adjacent to one side of the membrane region, and a second solvent region representing a solvent adjacent to another side of the membrane region;

a trained model prediction section configured to generate a second membrane permeability value expressing a membrane permeability of the peptide by extracting from the peptide a predictive feature vector expressing a feature and inputting the predictive feature vector into a trained model previously subjected to machine learning; and

a computation section configured to compute a predicted value of membrane permeability of the peptide by consolidating the first membrane permeability value generated by the simulation section with the second membrane permeability value generated by the trained model prediction section.

12. A trained model generation device comprising:

a simulation section configured to generate a predicted value of membrane permeability expressing membrane permeability of a peptide by simulating dynamics of the peptide permeating through a membrane region representing a cell membrane, a first solvent region representing a solvent adjacent to one side of the membrane region, and a second solvent region representing a solvent adjacent to another side of the membrane region; and

a training data generation section configured to generate simulation-derived training data expressed by the predicted value of peptide membrane permeability obtained by the simulation section paired with a feature vector generated from a 3D descriptor obtained from a tertiary structure of the peptide at each location; and

a training section configured to generate a trained model, for outputting a predicted value of the membrane permeability from the feature vector, by executing a machine learning algorithm based on training data including the simulation-derived training data generated by the training data generation section.

13. A trained model generation device comprising:

a training extraction section configured to extract a first training feature vector expressing a feature from each of a plurality of training cyclic peptides;

a training data generation section configured to generate a plurality of second training feature vectors for each of the first training feature vectors extracted by the training extraction section by cyclically shifting elements of the first training feature vectors, and to generate training data expressed by the first training feature vector and the plurality of second training feature vectors paired with a correct value for membrane permeability of the respective training cyclic peptide; and

a training section configured to generate a trained model, for outputting a predicted value of membrane permeability of a cyclic peptide from a feature vector expressing a feature of the cyclic peptide, by executing a machine learning algorithm based on a plurality of training data generated by the training data generation section.

14. A prediction device comprising:

an extraction section configured to extract a predictive feature vector expressing a feature from a cyclic peptide that is a target for membrane permeability prediction; and

a generation section configured to generate a predicted value of membrane permeability of the prediction target cyclic peptide by inputting the predictive feature vector extracted by the extraction section into the trained model generated by the trained model generation device of claim 13.

15. A trained model generation device comprising:
a training section configured to generate a trained convolutional neural network model for outputting a predicted value of membrane permeability of a cyclic peptide from a feature vector expressing a feature of a cyclic peptide by executing a machine learning algorithm based on training data expressed by a training feature vector expressing a feature extracted from each of a plurality of training cyclic peptides paired with a respective correct value of membrane permeability of the plurality of training cyclic peptides, the machine learning algorithm employing a convolutional neural network model including a both-end-adjacency layer in which elements at both ends of the training feature vector are placed adjacent to one another.

16. A prediction device comprising:

an extraction section configured to extract a predictive feature vector expressing a feature from a cyclic peptide that is a target for membrane permeability prediction; and

a generation section configured to generate a predicted value of membrane permeability of the cyclic peptide that is the target for membrane permeability by inputting the predictive feature vector extracted by the extraction section into a trained convolutional neural network model for outputting a predicted value of membrane permeability of a peptide from the feature vector, the trained convolutional neural network model including a both-end-adjacency layer in which elements at both ends of a feature vector expressing a feature of a cyclic peptide are placed adjacent to one another.

**17.** A prediction method, comprising:
by a computer:

extracting a predictive feature vector expressing a feature from a peptide that is a target for membrane permeability prediction;
adjusting a length of the extracted predictive feature vector to a prescribed length; and
generating a predicted value of membrane permeability of the prediction target peptide by inputting the predictive feature vector adjusted in length into a trained model pre-trained to output a predicted value of peptide membrane permeability from a feature vector expressing a feature of a peptide.

**18.** A trained model generation method, comprising:
by a computer:

extracting a training feature vector expressing a feature from each of a plurality of training peptides;
adjusting a length of each of the extracted training feature vectors for each of the plurality of training peptides to a prescribed length; and
generating a trained model, for outputting a predicted value of peptide membrane permeability from a feature vector expressing a feature of a peptide, by executing a machine learning algorithm based on training data that is the training feature vectors, adjusted in length, paired with correct values of membrane permeability of the training peptides.

**19.** A prediction method, comprising:
by a computer:

extracting each of predictive feature vectors expressing a feature, from a cyclic peptide that is a target for membrane permeability prediction for cases in which each of a plurality of residues contained in the cyclic peptide is at a start point of a cyclic sequence; and
generating a predicted value of membrane permeability of the prediction target cyclic peptide by inputting a plurality of the predictive feature vectors into a trained model pre-trained to output a predicted value of peptide membrane permeability from a feature vector expressing a feature of a cyclic peptide.

**20.** A trained model generation method, comprising:
by a computer:

extracting a training feature vector expressing a feature, from each of a plurality of training cyclic peptides for cases in which each of a plurality of residues contained in the training cyclic peptide is at a start point of a cyclic sequence; and
generating a trained model, for outputting a predicted value of cyclic peptide membrane permeability from a feature vector expressing a feature of a cyclic peptide, by executing a machine learning algorithm based on training data that is a plurality of extracted training feature vectors for each of a plurality of training cyclic peptides paired with a correct value of membrane permeability of the respective training cyclic peptide.

**21.** A prediction method to compute a predicted value of membrane permeability of a peptide permeating through a membrane region representing a cell membrane, a first solvent region representing a solvent adjacent to one side of the membrane region, and a second solvent region representing a solvent adjacent to another side of the membrane region, the prediction method comprising:
by a computer:

based on a result of simulation of a peptide permeating through the first solvent region, the membrane region, and the second solvent region, computing a free energy $G(z)$ of the peptide for each reaction coordinate $z$ expressing a position of the peptide in a region including the first solvent region, the membrane region, and the second solvent region and expressing a position of the peptide in a direction of an axis perpendicular to a membrane surface of the membrane region, and computing at each of the reaction coordinates z a difference $\Delta G(z)$ between a minimum value $G_{min}$ from among the free energies $G(z)$ of the peptide computed for the reaction coordinates z and the free energy $G(z)$ of the peptide at the reaction coordinate z;

computing a local diffusion coefficient $D(z)$ at each of the reaction coordinates z; and

computing a value $R(z)$ expressing a local resistance of the peptide at the reaction coordinate z based on the difference $\Delta G(z)$ computed for each of the reaction coordinates z and based on the local diffusion coefficient $D(z)$ computed for each of the reaction coordinates z, and computing a predicted value of membrane permeability of the peptide based on the value $R(z)$ expressing the local resistances computed for each of the reaction coordinates z.

22. A prediction method to simulate dynamics of a peptide permeating through a membrane region representing a cell membrane, a first solvent region representing a solvent adjacent to one side of the membrane region, and a second solvent region representing a solvent adjacent to another side of the membrane region, the prediction method comprising:

by a computer:

setting an initial conformation of the peptide according to relative substance permittivity in the first solvent region when simulating permeation of the peptide in a segment spanning from the first solvent region to a vicinity of a lipid molecule join positioned further toward the membrane center side than a boundary between the first solvent region and the membrane region; and

setting an initial conformation of the peptide according to relative substance permittivity in the membrane region when simulating permeation of the peptide in a segment spanning from the vicinity of the join to past a region of a membrane central zone representing a central area of the membrane region;

simulating dynamics of the peptide according to the set initial conformation of the peptide;

setting a series of initial conformations of each region in an umbrella sampling method based on a result obtained by simulation;

simulating the dynamics of the peptide according to an umbrella sampling method based on the series of initial conformations set for each region; and

predicting membrane permeability of the peptide based on a result of simulation based on an umbrella sampling method.

23. A prediction method to simulate dynamics of a peptide permeating through a membrane region representing a cell membrane, a first solvent region representing a solvent adjacent to one side of the membrane region, and a second solvent region representing a solvent adjacent to another side of the membrane region, the prediction method comprising:

by a computer:

when simulating permeation of the peptide using an umbrella sampling method, setting a spacing between restraint positions of the peptide so as to be finer the closer a region is to a membrane central zone representing a central area of the membrane region;

simulating dynamics of the peptide using an umbrella sampling method according to the spacing between the restraint positions; and

predicting membrane permeability of the peptide based on a result of the simulation.

24. A prediction method, comprising:

by a computer:

generating a first membrane permeability value expressing a membrane permeability of a peptide by simulating dynamics of the peptide permeating through a membrane region representing a cell membrane, a first solvent region representing a solvent adjacent to one side of the membrane region, and a second solvent region representing a solvent adjacent to another side of the membrane region;

generating a second membrane permeability value expressing a membrane permeability of the peptide by extracting from the peptide a predictive feature vector expressing a feature and inputting the predictive feature vector into a trained model previously subjected to machine learning; and

computing a predicted value of membrane permeability of the peptide by consolidating the generated first membrane permeability value with the generated second membrane permeability value.

25. A trained model generation method, comprising:

by a computer:

generating a predicted value of membrane permeability expressing membrane permeability of a peptide by simulating dynamics of the peptide permeating through a membrane region representing a cell membrane, a first solvent region representing a solvent adjacent to one side of the membrane region, and a second solvent region representing a solvent adjacent to another side of the membrane region;

generating simulation-derived training data expressed by the obtained predicted value of peptide membrane permeability paired with a feature vector generated from a 3D descriptor obtained from a tertiary structure of the peptide at each location; and

generating a trained model, for outputting a predicted value of the membrane permeability from the feature vector, by executing a machine learning algorithm based on training data including the generated simulation-derived training data.

**26.** A trained model generation method, comprising:

by a computer:

extracting a first training feature vector expressing a feature from each of a plurality of training cyclic peptides;

generating a plurality of second training feature vectors for each of the extracted first training feature vectors by cyclically shifting elements of the first training feature vectors, and generating training data expressed by the first training feature vector and the plurality of second training feature vectors paired with a correct value of membrane permeability of respective training cyclic peptides; and

generating a trained model, for outputting a predicted value of membrane permeability of a cyclic peptide from a feature vector expressing a feature of a cyclic peptide, by executing a machine learning algorithm based on a plurality of the generated training data.

**27.** A prediction method, comprising:

by a computer:

extracting a predictive feature vector expressing a feature from a cyclic peptide that is a target for membrane permeability prediction; and

generating a predicted value of membrane permeability of the prediction target cyclic peptide by inputting the predictive feature vector extracted by the extraction section into a trained model generated by the trained model generation method of claim 26.

**28.** A trained model generation method, comprising:

by a computer:

based on training data expressed by a training feature vector expressing a feature extracted from each of a plurality of training cyclic peptides paired with correct values of membrane permeability of the plurality of training cyclic peptides, generating a trained convolutional neural network model for outputting a predicted value of membrane permeability of a cyclic peptide from a feature vector expressing a feature of a cyclic peptide by executing a machine learning algorithm using a convolutional neural network model including a both-end-adjacency layer in which elements at both ends of the training feature vectors are placed adjacent to one another.

**29.** A prediction method, comprising:

by a computer:

extracting a predictive feature vector expressing a feature from a cyclic peptide that is a target for membrane permeability prediction; and

generating a predicted value of membrane permeability of the cyclic peptide that is the target for membrane permeability prediction by inputting the extracted predictive feature vector into a trained convolutional neural network model for outputting a predicted value of membrane permeability of a peptide from the feature vector, the trained convolutional neural network model including a both-end-adjacency layer in which elements at both ends of a feature vector expressing a feature of a cyclic peptide are placed adjacent to one another.

**30.** A non-transitory recording medium storing a prediction program executable by a computer to perform processing of the prediction method of any one of claim 17, claim 19, claim 21, claim 22, claim 23, claim 24, claim 27, and claim 29.

**31.** A non-transitory recording medium storing a trained model generation program executable by a computer to perform processing of the trained model generation method of any one of claim 18, claim 20, claim 25, claim 26, and claim 28.

# FIG.1

10

| 14 | 22 | 12 |
| TRAINING EXTRACTION SECTION | EXTRACTION SECTION | DATA STORAGE SECTION |

| 18 | 24 | 16 |
| TRAINING SECTION | GENERATION SECTION | TRAINING DATA STORAGE SECTION |

20

TRAINED MODEL STORAGE SECTION

# FIG.2

| DATA ID | TRAINING PEPTIDE INFORMATION | CORRECT VALUE FOR MEMBRANE PERMEABILITY OF TRAINING PEPTIDE |
|---|---|---|
| 00001 | Pe1 | Te1 |
| ... | ... | ... |
| ... | ... | ... |

# FIG.3A

# FIG.3B

RESIDUE 2 — RESIDUE 3

RESIDUE 1

RESIDUE 4

RESIDUE 8

RESIDUE 5

RESIDUE 7 — RESIDUE 6

# FIG.4A

| DATA ID | TRAINING FEATURE VECTOR | CORRECT VALUE FOR MEMBRANE PERMEABILITY OF TRAINING PEPTIDE |
|---------|-------------------------|-------------------------------------------------------------|
| 00001 | Fv1 | Te1 |
|  | Fv2 |  |
|  | ... |  |
|  | ... |  |
| ... | ... | ... |
| ... | ... | ... |

# FIG.4B

FEATURE
VECTOR

TRAINED
MODEL

PREDICTED VALUE
FOR MEMBRANE
PERMEABILITY
0.12

# FIG.5

# FIG.6

START

EXTRACT RESPECTIVE TRAINING FEATURE
VECTORS FOR INSTANCES IN WHICH EACH OF
PLURAL RESIDUES CONTAINED IN A
TRAINING CYCLIC PEPTIDE IS AT A START
POINT OF THE CYCLIC SEQUENCE — S100

GENERATE TRAINING DATA — S102

GENERATE A TRAINED MODEL BASED
ON PLURAL TRAINING DATA — S104

STORE TRAINED MODEL — S106

END

# FIG.7

```
                    ┌─────────┐
                    │  START  │
                    └─────────┘
                         │
                         ▼
┌──────────────────────────────────────────┐
│  RECEIVE PEPTIDE INFORMATION FOR A CYCLIC │         S200
│       PEPTIDE THAT IS THE TARGET FOR      │
│     MEMBRANE PERMEABILITY PREDICTION      │
└──────────────────────────────────────────┘
                         │
                         ▼
┌──────────────────────────────────────────┐
│    EXTRACT RESPECTIVE FEATURE VECTORS     │
│       FOR INSTANCES IN WHICH EACH OF      │         S202
│  PLURAL RESIDUES CONTAINED IN THE CYCLIC  │
│      PEPTIDE IS AT A START POINT OF       │
│           THE CYCLIC SEQUENCE             │
└──────────────────────────────────────────┘
                         │
                         ▼
┌──────────────────────────────────────────┐
│      GENERATE A PREDICTED VALUE OF        │
│  MEMBRANE PERMEABILITY BY INPUTTING       │         S204
│         THE PLURAL FEATURE                │
│    VECTORS INTO THE TRAINED MODEL         │
└──────────────────────────────────────────┘
                         │
                         ▼                           S206
┌──────────────────────────────────────────┐
│   GENERATE A REPRESENTATIVE VALUE FOR     │
│         MEMBRANE PERMEABILITY             │
└──────────────────────────────────────────┘
                         │
                         ▼                           S208
┌──────────────────────────────────────────┐
│             OUTPUT A RESULT               │
└──────────────────────────────────────────┘
                         │
                         ▼
                    ┌─────────┐
                    │   END   │
                    └─────────┘
```

# FIG.8

# FIG.9

```
              ┌─────────────┐
              │    START    │
              └──────┬──────┘
                     │
                     ▼
  ┌──────────────────────────────────────┐
  │    EXTRACT TRAINING FEATURE VECTORS   │────  S300
  │  FROM PLURAL TRAINING PEPTIDE INFORMATION │
  └──────────────────┬───────────────────┘
                     │
                     ▼
  ┌──────────────────────────────────────┐
  │  PERFORM ADJUSTMENT SUCH THAT LENGTHS │
  │   OF RESPECTIVE PLURAL TRAINING FEATURE│────  S302
  │    VECTORS BECOME PRESCRIBED LENGTH   │
  └──────────────────┬───────────────────┘
                     │
                     ▼
  ┌──────────────────────────────────────┐
  │       GENERATE TRAINING DATA          │────  S304
  └──────────────────┬───────────────────┘
                     │
                     ▼
  ┌──────────────────────────────────────┐
  │     GENERATE TRAINED MODEL BASED      │────  S306
  │      ON THE PLURAL TRAINING DATA      │
  └──────────────────┬───────────────────┘
                     │
                     ▼
  ┌──────────────────────────────────────┐
  │         STORE TRAINED MODEL           │────  S308
  └──────────────────┬───────────────────┘
                     │
                     ▼
              ┌─────────────┐
              │     END     │
              └─────────────┘
```

# FIG.10

```
        ┌─────────────┐
        │    START    │
        └──────┬──────┘
               │
               ▼
┌───────────────────────────────────┐
│    RECEIVE PEPTIDE INFORMATION FOR │ ──── S400
│ MEMBRANE PERMEABILITY PREDICTION TARGET │
└───────────────┬───────────────────┘
                │
                ▼
┌───────────────────────────────────┐
│      EXTRACT FEATURE VECTORS FROM  │ ──── S402
│         PEPTIDE INFORMATION        │
└───────────────┬───────────────────┘
                │
                ▼
┌───────────────────────────────────┐
│    PERFORM ADJUSTMENT SUCH THAT    │
│        LENGTHS OF FEATURE          │ ──── S404
│  VECTORS BECOME PRESCRIBED LENGTH  │
└───────────────┬───────────────────┘
                │
                ▼
┌───────────────────────────────────┐
│ GENERATE A PREDICTED VALUE OF MEMBRANE │
│    PERMEABILITY BY INPUTTING       │
│        LENGTH-ADJUSTED             │ ──── S406
│  FEATURE VECTOR INTO TRAINED MODEL │
└───────────────┬───────────────────┘
                │
                ▼
┌───────────────────────────────────┐
│           OUTPUT RESULT            │ ──── S408
└───────────────┬───────────────────┘
                │
                ▼
        ┌─────────────┐
        │     END     │
        └─────────────┘
```

# FIG.11

310

| 14 | 22 | 12 |
|---|---|---|
| TRAINING EXTRACTION SECTION | EXTRACTION SECTION | DATA STORAGE SECTION |

| 315 | 24 | 16 |
|---|---|---|
| TRAINING DATA GENERATION SECTION | GENERATION SECTION | TRAINING DATA STORAGE SECTION |

| 18 | 20 |
|---|---|
| TRAINING SECTION | TRAINED MODEL STORAGE SECTION |

# FIG.12

| 1 | 2 | 3 | 4 | 5 | 6 |
|---|---|---|---|---|---|
| A | B | C | D | E | F |
| B | C | D | E | F | A |
| C | D | E | F | A | B |
| D | E | F | A | B | C |
| E | F | A | B | C | D |
| F | A | B | C | D | E |

FIRST TRAINING FEATURE VECTOR

SECOND TRAINING FEATURE VECTORS

# FIG.13

CNN1

| 0 | A | B | C | 0 |
|---|---|---|---|---|

I

| 0AB | ABC | BC0 |
|-----|-----|-----|

Cv

# FIG.14

CNN2

| 0 | A | B | C | 0 |
|---|---|---|---|---|

I

| 0 | C | A | B | C | A | 0 |
|---|---|---|---|---|---|---|

I'

| 0CA | CAB | ABC | BCA | CA0 |
|---|---|---|---|---|

Cv

# FIG.15

510

32

SETTING
SECTION

31

SIMULATION
DATA STORAGE
SECTION

34

ENERGY
COMPUTATION
SECTION

33

SIMULATION
SECTION

35

DIFFUSION
COEFFICIENT
COMPUTATION
SECTION

36

PREDICTION
SECTION

# FIG.16

# FIG.17

# FIG.18

# FIG.19

# FIG.20

START

| | |
|---|---|
| SET INITIAL CONFORMATION | S500 |
| EXECUTE SIMULATION FOR OBTAINING SERIES OF INITIAL CONFORMATIONS | S502 |
| SET SERIES OF INITIAL CONFORMATIONS | S504 |
| SET RESTRAINT POSITIONS | S506 |
| EXECUTE REUS SIMULATION | S508 |
| COMPUTE FREE ENERGY G(z) | S510 |
| COMPUTE $\Delta$ G(z) | S512 |
| EXECUTE US SIMULATION | S514 |
| COMPUTE DIFFUSION COEFFICIENT D(z) | S516 |
| COMPUTE VALUE R(z) EXPRESSING LOCAL RESISTANCE | S518 |
| COMPUTE PREDICTED VALUE FOR PEPTIDE MEMBRANE PERMEABILITY | S520 |
| OUTPUT RESULT | S522 |

END

# FIG.21

610

40

SIMULATION
SECTION

44

TRAINED MODEL
PREDICTION
SECTION

42

TRAINED MODEL
STORAGE
SECTION

46

COMPUTATION
SECTION

# FIG.22

710

40

SIMULATION
SECTION

741

SIMULATION
RESULT
STORAGE
SECTION

715

TRAINING DATA
GENERATION
SECTION

716

TRAINING DATA
STORAGE
SECTION

718

TRAINING
SECTION

720

TRAINED MODEL
STORAGE
SECTION

Europäisches
Patentamt

European
Patent Office

Office européen
des brevets

## PARTIAL EUROPEAN SEARCH REPORT

under Rule 62a and/or 63 of the European Patent Convention.
This report shall be considered, for the purposes of
subsequent proceedings, as the European search report

**Application Number**

**EP 22 15 1777**

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | REZAI TAHA ET AL: "Conformational Flexibility, Internal Hydrogen Bonding, and Passive Membrane Permeability:? Successful in Silico Prediction of the Relative Permeabilities of Cyclic Peptides", JOURNAL OF THE AMERICAN CHEMICAL SOCIETY, [Online] vol. 128, no. 43, 6 October 2006 (2006-10-06), pages 14073-14080, XP055939365, ISSN: 0002-7863, DOI: 10.1021/ja063076p Retrieved from the Internet: URL:https://pubs.acs.org/doi/pdf/10.1021/ja063076p> [retrieved on 2022-07-05] * the whole document * * in particular * * "methods" * ----- | 7,8,21, 30 | INV. G16B15/30 G16B40/20 G16C20/30 G16C20/50 G16C20/70 |
| | -/-- | | TECHNICAL FIELDS SEARCHED (IPC) G16B G16C |

## INCOMPLETE SEARCH

The Search Division considers that the present application, or one or more of its claims, does/do
not comply with the EPC so that only a partial search (R.62a, 63) has been carried out.

Claims searched completely :

Claims searched incompletely :

Claims not searched :

Reason for the limitation of the search:

**see sheet C**

| Place of search | Date of completion of the search | Examiner |
|---|---|---|
| Munich | 2 November 2022 | Rákossy, Z |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04E07)

Europäisches
Patentamt

European
Patent Office

Office européen
des brevets

**PARTIAL EUROPEAN SEARCH REPORT**     Application Number

**EP 22 15 1777**

| | DOCUMENTS CONSIDERED TO BE RELEVANT | | CLASSIFICATION OF THE APPLICATION  (IPC) |
|---|---|---|---|
| Category | Citation of document with indication, where appropriate, of relevant passages | Relevant to claim | |
| A | YANG XIN ET AL:  "Concepts of Artificial Intelligence for Computer-Assisted Drug Discovery", CHEMICAL REVIEWS, [Online] vol. 119, no. 18, 25 September 2019 (2019-09-25), pages 10520-10594, XP055848230, US ISSN: 0009-2665, DOI: 10.1021/acs.chemrev.8b00728 Retrieved from the Internet: URL:https://pubs.acs.org/doi/pdf/10.1021/acs.chemrev.8b00728> [retrieved on 2022-07-04] * the whole document * * in particular * * section 6.1.3 * ----- | 7,8,21, 30 | |
| Y | LEUNG SIEGFRIED S. F. ET AL:  "Testing Physical Models of Passive Membrane Permeation", JOURNAL OF CHEMICAL INFORMATION AND MODELING, [Online] vol. 52, no. 6, 25 June 2012 (2012-06-25), pages 1621-1636, XP055939382, US ISSN: 1549-9596, DOI: 10.1021/ci200583t Retrieved from the Internet: URL:https://www.ncbi.nlm.nih.gov/pmc/articles/PMC3383340/pdf/nihms380051.pdf> [retrieved on 2022-07-05] * the whole document * * in particular * * theory section * ----- -/-- | 7,8,21, 30 | TECHNICAL FIELDS SEARCHED     (IPC) |

Europäisches
Patentamt

European
Patent Office

Office européen
des brevets

**PARTIAL EUROPEAN SEARCH REPORT**

Application Number

**EP 22 15 1777**

| | DOCUMENTS CONSIDERED TO BE RELEVANT | | CLASSIFICATION OF THE APPLICATION (IPC) |
|---|---|---|---|
| Category | Citation of document with indication, where appropriate, of relevant passages | Relevant to claim | |
| A | GENG HAO ET AL: "Applications of Molecular Dynamics Simulation in Structure Prediction of Peptides and Proteins", COMPUTATIONAL AND STRUCTURAL BIOTECHNOLOGY JOURNAL, vol. 17, 26 July 2019 (2019-07-26), pages 1162-1170, XP055938766, Sweden ISSN: 2001-0370, DOI: 10.1016/j.csbj.2019.07.010 * the whole document * * in particular * * page 1167 and sections 2 and 3 * ----- | 7,8,21, 30 | |
| | | | **TECHNICAL FIELDS SEARCHED (IPC)** |

EPO FORM 1503 03.82 (P04C10)

Europäisches
Patentamt
European
Patent Office
Office européen
des brevets

**INCOMPLETE SEARCH
SHEET C**

Application Number

**EP 22 15 1777**

```
Claim(s) completely searchable:
      7, 8, 21

Claim(s) searched incompletely:
      30

Claim(s) not searched:
      1-6, 9-20, 22-29, 31

Reason for the limitation of the search:

Claims 1, 3, 4, 6, 7, 9, 10, 11, 12, 13, 15, 16, 17, 18, 19, 20, 21, 22,
23, 24, 25, 26, 27, 28, 29, 30, 31 have been drafted as separate
independent claims.
In reply to the invitation pursuant to Rule 62a(1) EPC, the applicant has
specified that claim 7 and corresponding method claim 21 and medium claim
30 should be searched. The applicant has also filed an amended set of
claims, with a request to search amended claims. No amended claim set can
be submitted at this stage of the procedure, hence the request to search
any amended claims cannot be granted. Hence this ESOP refers to the
specified claims in the reply which are part of originally submitted
claims, namely claims 7, 8, 21 and 30.
The applicant's attention is drawn to the fact that the application will
be further prosecuted on the basis of subject-matter for which a search
has been carried out and that the claims should be limited to that
subject-matter at a later stage of the proceedings (Rule 62a(2) EPC).
```

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2017037378 A
- WO 2003054743 A
- JP 2020523010 A
- JP 2020519246 A
- JP 2021031234 A

**Non-patent literature cited in the description**

- **SIEWERT-JAN MARRINK ; ERMAN J. C. BERENDSEN.** Simulation of water transport through a lipid membrane. *J. Phys. Chem.,* 1994, vol. 98 (15), 4155-4168
- **YUJI SUGITA ; AKIO KITAO ; YUKO OKAMOTO.** Multidimensional replica-exchange method for free-energy calculations. *J. Chem. Phys.,* 2000, vol. 113 (15), 6042-6051
- **ALESSANDRO LAIO ; MICHELE PARRINELLO.** Escaping free-energy minima. *Proc. Natl. Acad. Sci.,* 2002, vol. 99, 12562-12566